**Europäisches Patentamt**

**European Patent Office**

**Office européen des brevets**

(19)

(11) **EP 1 452 993 A1**

(12) **EUROPEAN PATENT APPLICATION**

(43) Date of publication:
**01.09.2004 Bulletin 2004/36**

(51) Int Cl.$^7$: **G06F 19/00**

(21) Application number: **02425791.7**

(22) Date of filing: **23.12.2002**

| | |
|---|---|
| (84) Designated Contracting States:<br>**AT BE BG CH CY CZ DE DK EE ES FI FR GB GR IE IT LI LU MC NL PT SE SI SK TR**<br>Designated Extension States:<br>**AL LT LV MK RO**<br><br>(71) Applicant: **STMicroelectronics S.r.l.**<br>**20041 Agrate Brianza (Milano) (IT)**<br><br>(72) Inventors:<br>• **Alessi, Enrico**<br>**95127 Catania (IT)** | • **Oliveri, Salvatore**<br>**95022 Aci Catena (IT)**<br>• **Di Francesco, Flavio**<br>**93012 Gela (IT)**<br>• **Licciardello, Antonella**<br>**95045 Misterbianco (IT)**<br><br>(74) Representative: **Pellegri, Alberto et al**<br>**c/o Società Italiana Brevetti S.p.A.**<br>**Piazza Repubblica, 5**<br>**21100 Varese (IT)** |

(54) **Method of analysis of a table of data relating to expressions of genes and relative identification system of co-expressed and co-regulated groups of genes**

(57)    A method for automatic analysis of the genomic information, in order to determine relationships among genes involved in a same regulating process allows to determine complex relationships among genes that go beyond the simple clustering operations of known methods aimed to determine co-expressed or co-regulated genes.

First a clustering algorithm is chosen and is applied to the table, obtaining sub-tables of data relative to groups of genes that satisfy the chosen clustering criterion. Therefore, all possible combinations of pair of sub-tables are generated and characteristic parameters are calculated for genes contained in these sub-tables. Finally, for each combination a characteristic value is calculated with a decision algorithm defined in function of these parameters, by considering the genes of the combination as constituting a 'Gene Network' if this characteristic value exceeds a pre-defined threshold.

The method is preferably is implemented by a relative system of identification of groups of co-expressed and co-regulated genes comprising an intelligent fuzzy sub-system trained off-line identified by a neural network.

**FIG. 4**

**Description**

FIELD OF THE INVENTION

The present invention generally relates to genomic analysis and more in particular to a method and a relative identification system of co-expressed and co-regulated groups of genes from the analysis of data relative to gene expression varying with time or relative to different conditions.

BACKGROUND OF THE INVENTION

The knowledge of the sequence of the human and of other organisms genome provides to researchers fundamental tools for developing strategies for preventing and curing diseases that in the majority of cases are considered to be caused by the simultaneous action of different genes.

All the cells of an organism contain the same genetic characteristics, but the number of genes that are expressed, that is copied in mRNA, is specific for each type of cell; in this sense the mRNA reveals the active genes. Each cell of our body expresses only a specific set of genes according to a highly regulated program that confers to that cell its distinctive pattern and its functional capabilities. The gene expression program of a genome defines the role and the behavior of every cell of an organism.

The gene information of each cell controls that proteins are synthesized, and in which amount, through the precise control of the genic expression of the components transcribed from each gene *(Regulation).*

The so-called Gene-Chips are extraordinary tools for studying patterns of genic expression, intended as the collection of activity states of a set of genes.

A comparative study of gene expression of different tissues, or at the same time at different development states, allows to understand which genes characterize a cellular type from another and how the various cellular types differ from one another.

Thanks to these technologies, nowadays there is a large amount of data relating to the level of gene expression is available.

An objective of the genomic research is the interpretation of patterns of genic expression, focusing on which the interactions gene-gene among genes that are involved in a same regulating process may be. Given that each experiment generates a large amount of data, they must be organized using "Data Mining" techniques.

The term "Data Mining", cited in literature also as "Knowledge Discovery in Database", indicates all the numerous techniques for extracting information that is potentially useful and not yet known from a set of data.

Known Data Mining techniques are those based on clustering algorithms that may be classified in two different types:

- semantic clustering, based on semantic properties of a certain entity;
- numerical clustering, based on quantitative properties of a certain entity.

While according to the classic approaches a gene or a sequence at the time was studied, the genomic research is nowadays directed towards the development of technologies that allow a parallel analysis of many thousands of genes at the same time. The state of the art in this field relates to the construction of matrices (microarrays) with nucleic acids for studying the mRNA level of biological samples.

The present state of the art in the field of the analysis of genomic information is characterized by the use of exclusive and not supervised clustering techniques. Through these techniques, the gene sequences, which are represented by a vector whose elements are the time expressions of a gene determined in a specific biological contest, or more generally the values of expression under different conditions, are grouped together by similarity with algorithms that do no require any prior knowledge.

The term "clustering" is thus used, in this specific context, for indicating clustering criteria for partitioning a table of data relative to genes in sub-tables containing data of genes that have similar behavior. In practice, by using the clustering criteria it is possible to partition gene sequences according to characteristics of similarity in time evolution of the expression or in comparing different states.

A brief presentation of known clustering criteria is carried out in the following paragraph.

DATA MINING

Data relative to expression of examined genes, are organized, kept and updated in a table, as Table 1. In the columns are reported levels of expressions at different time instants or for different initial conditions of the cycle of experiments.

The set of the levels of expression of a gene at the different time instants (data of a same row) is called *temporal profile of gene expression.*

The column ORF[1] contains alphanumeric gene identification values, called also "accession numbers", that specify to which genes they refer in the external websites that are visited for a research. Each time a new gene is discovered, it receives an "accession number" for storing it in public databases.

The cells of the table contain information of a specific gene correlated with its sample. The value "5.8" of the first row and of the fourth column of Table 1, indicates that the value or level of gene expression, observed for the gene YAL001C after two hours from the first experiment, is 5.8. A void cell indicates absence of information.

Given a set of genes, the set of their activity states, intended as levels of expression, at a certain instant (data of a same column), is called *pattern of genic expression.*

At the present time, the most used computing technique for analyzing genic expressions is the numerical clustering of genes based on the similarity of expression patterns.

In the field of bioinformatics, clustering techniques generate groups of genes (CLUSTERS) for:

- extracting "regulating motifs"[2]
- finding functional families;
- classifying types of cells, samples of tissue etc.

**Extraction of regulating motifs**

*Should the sequence of a gene be known, such as in the case of the yeast, it is possible to identify the regulating motifs of a CLUSTER of genes that present similar levels of gene expression.* In other words, it is possible to deduce the co-regulation from the co-expression: it is probable that co-expressed genes, belonging to the same CLUSTER, have common regulating motifs.

The CLUSTERS, obtained by grouping time sequences of similar genic expressions, may be analyzed for predicting the transcription factors that are responsible of the synthesis of a protein.

**Deduction of functional families**

Another objective of the clustering techniques is that of identifying genes that have similar functions, i.e. that are involved in a same cellular process. If, for example, an unknown gene is grouped with a number of genes whose functional family is known (by means of the semantic clustering), it is possible to deduce the function of the examined gene.

**Classification of types of cells, sample of tissue etc.**

Another way of using gene expression data is to classify them, by identifying and distinguishing among different types of cells, sample of tissue etc.

It is supposed that genes belonging to a same group (CLUSTER) concur to form a same kind of cell or of a same tissue.

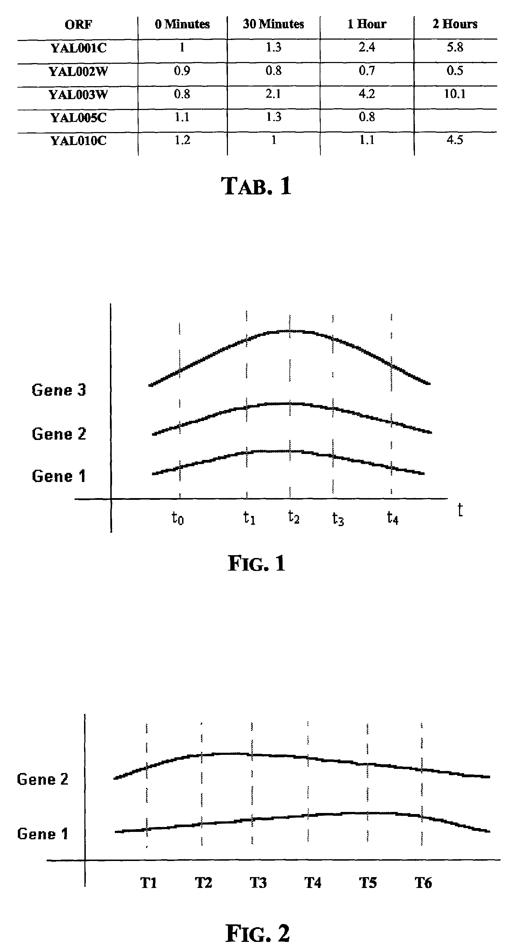

The information obtained with Data Mining techniques may be interpreted with the help of graphs such as those of Figures 1 and 2. The time diagrams of genes in the graph of Figure 1 are referred to a same sample (profiles of time expression).

As it may be noticed, the genes 1 and 2 have similar characteristics, because both of them have a similar time evolution (very close values of profiles of expression). It must be expected that both these genes will react to the same internal or external stimuli: for instance, their activity could be influenced by the same set of inputs, that is by the same set of regulating genes.

Differently, the diagram of Figure 2 compares levels of genic expressions at a fixed time instant relative to different samples. This comparison helps in understanding whether a gene (maybe together with other genes) could be responsible of a certain disease.

For example, if a sample (T5) of a symptomless carrier of a disease, such as diabetes, has a gene (Gene1) with a level of expression that shows a large variation in respect to the value of the same examined gene from other samples of people not affected by diabetes, it would be possible to suppose that Gene1 be the cause of the disease.

Clustering techniques, besides being a powerful tool for analyzing the gene expression, are used in the so-called pre-processing phase of a dynamic regulating gene network or Gene Network. In order to model such a Gene Network, it is necessary to know first which and how many genes constitute the network. In the pre-processing phase

---

[1] Open Reading Frame
[2] Short DNA sequences that are tied to the RNA polymerase defining a starting point of the transcription.

are selected one or more groups of genes that may constitute a Gene Network.

Because of the importance of clustering techniques, in the following paragraphs these techniques of Data Mining are presented.

**Numerical clustering**

A numerical clustering technique uses mathematical algorithms for grouping genes based on the similarity of obtained values of genic expression.

Briefly, a numerical clustering technique is constituted by two parts: a *measure of distance* that indicates how much the patterns of expression of a pair of genes (or more generally of two clusters) are similar and a clustering algorithm for identifying CLUSTERS of similar patterns of genic expression based on the chosen measure of distance.

**Semantic clustering**

The sequencing of the human genome and of many other sample organisms in the last years gave a particular importance to that sector of the bioinformatics that studies DNA and proteins. Thanks to the introduction of methods of automatic sequencing the DNA and to the numerous projects of gene sequencing, the amount of information on proteic sequences has hugely increased. The great amount of data so produced needs to be collected, stored and distributed.

Given that the managing such an amount of data requires an intensive use of computers, the development of databases is one of the main points of the genome program. Databases must be designed accurately and their architecture must be such to store information about the map (for example the physical location of a gene) about the (nucleotide and protein) sequences and they must also provide links to databases storing scientific and medical information.

Nowadays, through Internet, many databases are accessible in which laboratories of genetics of all over the world daily upload the obtained data, while developing also tools for the analysis and comparison of this information. The largest public databases that store nucleotidic sequences are: GenBank (http://www.ncbi.nlm.nih.gov/), EMBL (European Molecular Biology Laboratory, http://www.ebi.ac.uk/Information/index.html) and DDBJ (DNA Database of Japan, http://www.ddbj.nig.ac.jp). The largest databases that store protein sequences are: PIR (Protein Identification Resource - National Biomedical Research Foundation), Swissprot and GenPept (both distributed with GenBank). Together with information about sequences, they contain information about regulating sequences of proteins and about other features of the structure of proteins.

In general, these databases contain information about known gene sequences, such as for example information concerning the "functional domains" or attributes (the so-called "ontologies") of a specific product of the gene. Examples of attributes of a gene product are the *molecular function*[3], *the biological process*[4] and the *cellular component*[5].

These attributes of a gene are parameters of semantic nature, that is they are expressed by words chosen in a specific vocabulary. The realization of this vocabulary is an objective of the Gene Ontology consortium (http://genome-www.stanford.edu/saccharomyces/help/GO.html, http://www.geneontology.org).

By associating to these semantic parameters corresponding numerical values it is possible to apply known clustering techniques for grouping genes that share similar semantic characteristics. In this way CLUSTERS of genes presenting similar functional domains or attributes (ontologies) are obtained.

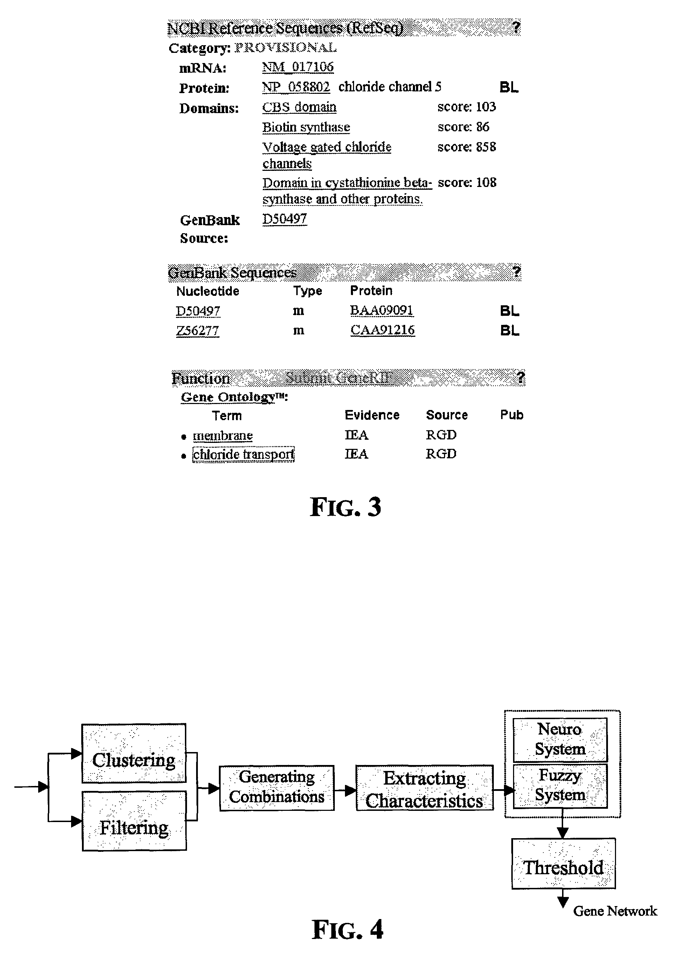

Figure 3 depicts a sample report obtained by making a query at the website LocusLink (http://www.ncbi.nlm.nih.gov/LocusLink/). The query has been made by inserting the accession number (univocal identification string for the specified database) of the considered gene (in the depicted example the accession number is D50497). Among the results of the query there are also ontologies, indicated in the section in which there is the trademark Gene Ontology™ (trademark of the Gene Ontology Consortium).

In order to better understand the meaning of the expression "functional domain", a brief discussion on the structure of proteins that constitute the DNA is presented hereinafter.

THE STRUCTURE OF PROTEINS

In proteins at the native state, polypeptide chains do not have a disordered or random 3D structure, but on the contrary for each protein they are disposed in the same way and are presented as objects with an identical shape

[3] The term *molecular function* indicates the capacity that a gene potentially has, the biochemical activity of the gene product, what it can do without specifying where or when this happens. Examples of such terms are "enzyme", "transporter", "ligand", "adenylate cyclase" or "Toll receptor ligand".
[4] The term *biological process* refers to the biological objective to which the gene or gene product gives its contribution. Examples of such terms are "cell growth and maintenance", "signal transduction", "pyrimidine metabolism" or "cAMP biosynthesis".
[5] The term *cellular component* refers to the site in the cell in which a product of a gene is active.

(structure) in all the molecules of a certain protein. This depends on the fact that each molecule, inserted in a determined environment, assumes a disposition in the space that allows to establish the maximum possible number of interactions both among atoms or groups of atoms that are part of the same molecule, and among atoms or group of atoms of the neighboring molecules.

Different levels of structural complexity are described by a primary structure, a secondary structure, a tertiary structure and, in certain cases, a quaternary structure of proteins. It must be remarked that this is a simplification for describing proteins, because the different levels of structural complexity constitute the specific 3D structure of each protein.

The succession of amino-acids is called primary structure and it indicates exactly the covalent structure of the peptide chain. Each protein is characterized by its own specific sequence of amino-acids, different from that of any other protein. Therefore the DNA contains coded information relating to the primary structure of all proteins.

It is fundamental to know even as the peptide chains are spatially disposed and associated for comprehending how a protein performs certain functions.

The tertiary structure, which represents the true 3D structure of the protein, varies from a protein to another and consists in the mode in which the chain, partially organized in secondary structure, is coiled to generate the native protein. The portions of chains in secondary structures are connected by irregular tracts that form loops, that in certain cases have a certain mobility. The coiling is not random and is strongly conditioned by the environment in which the protein is. The fact that the polypeptide chain is coiled implies that lateral chains of amino-acids located in far points of the primary structure become close.

These "sub-coils" are called "structural domains" of the protein. Often they keep their structure even when they are separated by the rest of the protein.

Sometimes *structural domains* perform a specific sub-function in the biological function of proteins (for example, in certain enzymes a domain operates as a catalyst, while another domain interacts with the substances that regulate the activity of the enzyme): in these cases, a relative structural autonomy of part of the polypeptide chain is associated with a relative functional autonomy. These structural domains are called *functional domains* of the protein. It is important to notice that in a number of proteins, that perform partially similar functions, there are similar domains that perform the same function in all these proteins.

The most diffused and used clustering algorithms are the agglomerative hierarchic clustering algorithm, the K-means and the SOM (Self Organizing Map) algorithms. The results of the used clustering algorithms depend on the metric used for defining the similarity criterion among gene sequences. As a consequence, two gene sequences that have been judged similar using a certain metric, may be judged different using another metric.

Actually the gene research is limited to the use of clustering algorithms for identifying gene sequences that behaves in a similar mode in a certain biological process.

A limit of the known identification techniques of gene networks is represented by the fact that it is not possible to identify groups or sequences of genes by applying at the same time a plurality of properly weighed criteria for determining sequences that may be even different among them from the point of view of the gene expression with time, but that have specific properties of particular interest.


OBJECT AND SUMMARY OF THE INVENTION


It has been found and is the object of the present invention a method for automatic analysis of the genomic information, in order to determine relationships among genes involved in a same regulating process. The method of the invention allows to determine complex relationships among genes that go beyond the simple clustering operations of known methods aimed to determine co-expressed or co-regulated genes.

The method of the invention is applicable to a table of data relative to the evolution of the gene expression with time or relative to different stress conditions, and does not depend on the method used for obtaining this table.

First a clustering algorithm is chosen and is applied to the table, obtaining sub-tables of data relative to groups of genes (CLUSTERS).

Therefore, all possible combinations of pair of sub-tables are generated and characteristic parameters are calculated for genes contained in these sub-tables.

Finally, for each combination a characteristic value is calculated with a decision algorithm defined in function of these parameters, by considering the genes of the combination as constituting a 'Gene Network' if this characteristic value exceeds a pre-defined threshold.

Preferably, also a group of logic filtering criteria of data of the table is chosen, generating other sub-tables of data of groups of genes that satisfy the respective logic criterion, and combinations among pairs of sub-tables, obtained with logic or clustering criteria, are calculated.

Preferably, the decision algorithm is a fuzzy algorithm whose antecedents and consequents are defined in function of these characteristic parameters.

The method of this invention is implemented by a relative system of identification of groups of co-expressed and co-regulated genes. The core of this identification system is an intelligent sub-system that processes the characteristic parameters of groups of genes and outputs data of groups of genes identified as 'Gene Networks'.

Preferably, this intelligent sub-system is an fuzzy sub-system trained off-line identified by a neural network.

The invention is more precisely defined in the annexed claims.

BRIEF DESCRIPTION OF THE DRAWINGS

The different aspects and advantages of the invention will appear even more evident through a detailed description referring to the attached drawings, wherein:

**Table 1** is sample table of data relating to gene expressions;

**Figure 1** is a diagram of levels of gene expression at different instants relating to the same sample of DNA;

**Figure 2** is an interpolated diagram of the levels of genic expression at a certain pre-established instant, relative to different samples (T1, ..., T6) of DNA;

**Figure 3** is an example of a report obtained carrying out a query at the website LocusLink;

**Figure 4** depicts a preferred embodiment of a system of the invention;

**Figure 5** shows sample scatter diagrams;

**Figure 6** shows examples of diagrams of data correlated according to a quadratic law;

**Figure 7** shows possible time evolutions of gene sequences;

**Figure 8** shows a set of data for training the Fuzzy system of the invention;

**Table 2** contains a set of values of gene expressions of the yeast S. *cerevisiae* in different instants;

**Table 3** contains data extracted from the Saccharomyces Genome Database for several genes mentioned in Table 2;

**Table 4** contains data relating to several genes of Table 2 that have been grouped in a CLUSTER;

**Table 5** shows possible combinations between two groups of genes and the characteristic value associated to each combination;

**Tables 6 and** 7 show data relative to genes grouped in CLUSTERS 26 and 30;

**Table 8** shows levels of genic expression of the combination among CLUSTERS of Tables 6 and 7;

**Table 9** shows levels of expression of Table 8 normalized to range between 0 and 1;

**Table 10** shows values of increments of levels of expression of Table 9.

DESCRIPTION OF SEVERAL EMBODIMENTS OF THE INVENTION

The method of the present invention allows to identify groups of genes ('Gene Networks') that are likely to be involved in a regulating process. This method is based on a decision algorithm that, differently from known methods, identifies groups of co-expressed or co-regulated genes using both clustering criteria and logic filtering criteria. From each group of genes characteristic parameters are calculated and, with a decision algorithm based on these characteristic parameters, a characteristic value is calculated: when this characteristic value exceeds a certain pre-established threshold, the relative group of genes is identified as a constituent of a 'Gene Network', otherwise is discarded.

The remarkable advantage of this technique consists in the fact that the limits of present methods based exclusively on clustering are overcome, and it allows to identify a group of genes as a 'Gene Network' according to differently combined criteria.

Preferably, this decision algorithm is a fuzzy algorithm configured such to identify correlations among genes within a large amount of data, corresponding to the time variable gene expression or relating to different conditions of gene sequences fixed on a microarray.

A schematic representation of a preferred embodiment of a system implementing the method of the invention is shown in Figure 4.

The system includes three sub-systems:

1. A pre-processing sub-system (CLUSTERING, FILTERING), that generates groups of tables using clustering criteria and logic filtering criteria.

2. A processing sub-system (GENERATING COMBINATIONS, EXTRACTING CHARACTERISTICS) that generates groups of genes that are Gene Networks candidates, by combining pairs of sub-tables and extracting characteristic parameters for each combination of genes.

3. An intelligent sub-system (NEURO SYSTEM, FUZZY SYSTEM, THRESHOLD), trained off-line, that output groups of genes identified as Gene Networks.

The intelligent sub-system is preferably a Fuzzy system, trained off-line, capable of attributing to each candidate group of genes a characteristic value using a soft computing decision making algorithm (see [22]): if this characteristic value exceeds a pre-established threshold THRESHOLD, the relative group of genes is identified as constituting a Gene Network.

**Clustering and Filtering**

The pre-processing sub-system generates similar groups of gene sequences using clustering criteria and logic filtering criteria. There is a plenty of clustering criteria in literature, as that cited hereinbelow:

- Hierarchical Agglomerative;
- Non hierarchical Kmeans;
- Hierarchical sequential Kmeans;
- Non hierarchical SOM;
- Non exclusive Fuzzy Clustering.

For each gene *m* values of gene expression are reported, relating to m experiments carried out in different instants or different conditions. The system generates a certain number of groups of genes (CLUSTERS) according to the criterion used and the initial settings chosen for the processing.

In order to study Gene Networks, it is interesting to select groups of genes that presents other characteristics, besides the similarity of time expression profiles. This is made possible through filtering techniques that select groups of genes, depending on the value assumed by one or more attributes of the gene itself.

The criteria to be used must be chosen considering whether or not they are appropriate for constituting groups of similar gene sequences. For example, when the influence of extended groups of genes among them and towards single genes must be verified, it is preferable to use a stringent logic filtering criterion and clustering algorithms that generate extended groups of CLUSTERS. Such a choice may consist in a single linkage hierarchical method coupled to the metric used for updating the matrix of distances.

A filtering criterion is any logic criterion that an user may impose on the data of a table. For example, a filtering criterion may consist in selecting all the genes whose level of expression exceeds a certain value at the beginning of the experiment. Another filtering criterion may be that of considering the genes whose level of expression after 7 minutes from the beginning of the experiment is comprised in a certain interval and after 14 minutes is comprised in another interval, and the like.

**Generation of combinations and composition of groups of genes candidate to be a Gene Network**

Let us suppose of having generated *K* sub-tables of genes (CLUSTER) with a certain clustering criterion and *M* sub-tables of genes (FILTER) with certain logic filtering criteria. According to the method of this invention, all possible groups of genes are generated by combining in pairs the sub-tables:

1.

$$\binom{K}{2} = \frac{K!}{(K-2)! * 2!} = \frac{K * (K-1)}{2}$$

CLUSTER-CLUSTER combinations;

2.

$$\binom{M}{2} = \frac{M!}{(M-2)! * 2!} = \frac{M * (M-1)}{2}$$

FILTER-FILTER combinations;

3. *K* * *M* CLUSTER-FILTER combinations.

Preferably, among these combinations, the ones that generate groups of genes with a number of genes smaller than a certain pre-established threshold are discarded together with the combinations that generate groups of genes already obtained in a previous combination.

Each gene of the combination may be labeled with a string that indicates the group it belongs to. For example, a gene is labeled C2 if the group it belongs to is the cluster 2. In a combination FILTER-FILTER or CLUSTER-FILTER, a gene present in both groups is labeled $F_iF_j$ or $C_iF_j$, where the subscripts *i* and j are the indexes of the CLUSTER or the FILTER the gene is coming from.

When the aim is to determine how the behavior of a certain gene influences a whole CLUSTER, it is preferable to generate combinations among a FILTER constituted only by that gene and groups (CLUSTERS) constituted by more genes.

**Extraction of characteristics**

The most significant phase is constituted by the extraction of characteristics because they indicate the type of correlations that must be identified. According to an innovative feature of the present invention, numerical parameters, tied to the gene expression profile, and parameters that, on the contrary, have a semantic meaning, or mixed parameters obtained as combination of both elements, are used. A sample semantic parameter is the numerical representation of functional domains of the homologous amino-acid sequence corresponding to the starting nucleotide sequence fixed on microarray.

A sample semantic parameter that is considered in Gene Network analysis is the percentage of genes of the combination with the same functional domain. A number ranging from 0 to 1 is associated to this percentage. When the value of the parameter is 1, all the genes of the considered combination have the same functional domain. When the value is null, the genes do not have any common domain, while in all other cases the value of this parameter is comprised between 0 and 1.

Similarly to the previous case, another semantic parameter relates to the percentage of genes the have equal ontologies or ontologies belonging to the same category. It is evident that other semantic parameters could be considered by extending this analysis to other semantic characteristics of gene sequences. It must be underlined that these parameters refer to semantic characteristics but are expressed in numerical form.

According to an effective embodiment of this invention, six numeric parameters P1, ..., P6 are used. Every parameter ranges between 0 and 1.

The first parameter P1 is the absolute value of the linear correlation coefficient among the expressions of pairs of genes of the same combination if the correlation is positive, otherwise it is null. The second parameter P2 is analogous to P1, but is null if the linear correlation is positive. The third parameter P3 indicates the value of the quadratic correlation of the combination. The more the value of the correlation is close to 1, the more the genes of the combination are correlated.

The fourth parameter P4 indicates the percentage of genes of the group whose value of final gene expression (that is the last attribute of the gene) is greater or smaller than the initial value of gene expression (first attribute). In practice, the percentage of genes that have the same global variation is calculated.

The fifth parameter P5 indicates the percentage of genes of the group that has the same time evolution (increasing or decreasing). Finally, the last parameter P6 indicates the percentage of genes that presents a peak excursion at the same instant.

The entering of these parameters is aimed to verify whether the group of examined genes is constituted by differently expressed genes that participate in a same regulating process and so whether the relationships among them may be modeled by a Gene Network.

*Using the above specified six parameters in order to determine groups of co-expressed and co-regulated genes provides a robust method of identification capable of a multi-objective discrimination.*

It must be reminded that this approach may be generalized to any parameter of interest that expresses a correlation of any kind among values of expression and genes.

Moreover, it is possible to use parameters that may have a completely semantic biological meaning or more complex mixed parameters that express at the same time a numerical and a semantic correlation. In the latter case, external databases to which a query may be submitted and the processing output data for obtaining a numerical codification that expresses the eventual semantic correlation.

A detailed examination of the meaning of the six proposed parameters is examined hereinafter.

**Parameters relating to correlation (P1, P2, P3)**

The correlation indicates the level of relationship among genes. Through it, it is possible to determine how a linear equation or any other equation is appropriate to describe or explain such a relationship.

Being $X$ and $Y$ two profiles of time evolutions or genic expressions to be examined, it is possible to make a *scatter diagram* in a system of Cartesian coordinates. Should all the points of the scatter diagram lay around a straight line, the correlation is linear. In this case the equation that ties the two variables is a linear equation

$$Y=a+bX \tag{1}$$

If $Y$ increases when $X$ increases, the correlation is said to be positive or direct. If $Y$ decreases when $X$ increases, the correlation is said to be negative or inverse. If there is not any linear relation between the two sequences, they are said to be *uncorrelated.* The degree of linear correlation between two gene sequences is given by the linear correlation coefficient defined as follows:

$$\rho = \frac{\sum (X - \bar{X})(Y - \bar{Y})}{\sqrt{\sum (X - \bar{X})^2 \, \Sigma (Y-\bar{Y})^2}} \quad -1 \le \rho \le 1 \tag{2}$$

wherein the sum goes from 1 to $m$ (being m the number of levels of expression calculated for each gene) and $\bar{X}=\frac{\Sigma X}{m}$ and $\bar{Y}=\frac{\Sigma Y}{m}$ are the mean values.

The linear correlation is maximum when the absolute value of the coefficient $\rho$ is equal to 1 (the sign depends on the fact that a variable increases or decreases when the other variable increases).

Figure 5 illustrates examples of scatter diagrams.

A null value of the linear correlation coefficient implies only the absence of a linear correlation, nevertheless two sequences may be strongly dependent from each other while not presenting a strong linear correlation. A typical case is that of points distributed along a circumference.

Sometimes, the correlation between two gene sequences may be of a quadratic type, that is the relationship between X and $Y$ is the equation of a parabola:

$$Y=a+bX+cX^2 \tag{3}$$

wherein $a$ is a constant, $b$ is the linear growth coefficient and $c$ is tied to the curvature and is due to the relation between $Y$ and the square power of $X.$

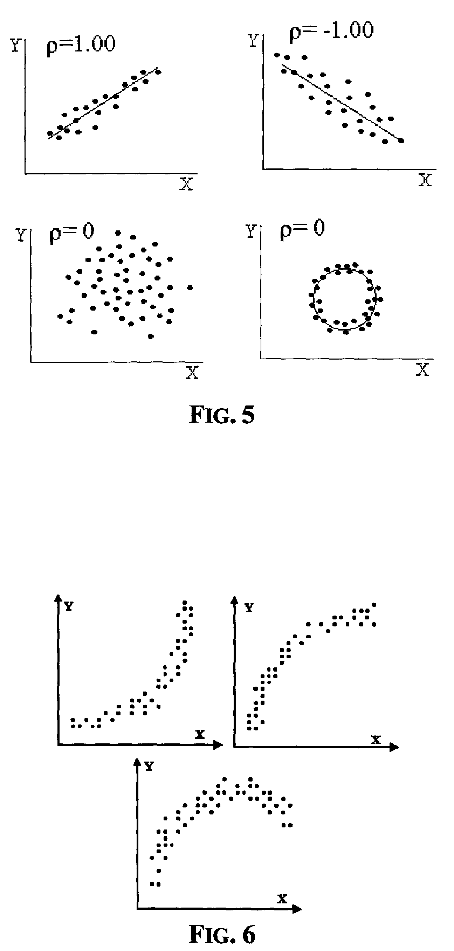

Figure 6 shows examples of quadratic correlation.

In general, whichever the relation between X and Y is, the correlation coefficient is defined as:

$$r=\pm \sqrt{\frac{\sum (Ystim - \bar{Y})^2}{\sum (Y-\bar{Y})^2}} \tag{4}$$

wherein *Ystim* is the interpolated value of Y obtained by the method of minimum squares. It is worth noticing that $r$ is a non dimensional quantity, that is it does not depend on the unity of measure. When the relation between $X$ and $Y$ is linear, $r$ coincides with the linear correlation coefficient, otherwise it has a more general meaning. Moreover, in the case in which the relation is linear,

$$r_{XY} = r_{YX}$$

that is the quantity $r$ is the same irrespectively from the fact that $X$ or $Y$ is the independent variable. In general

$$r_{XY} \neq r_{XY}$$

As said before, the first three extracted parameters relate to the linear and to the quadratic correlation. Let us consider one of the generated combinations and let us assume that the corresponding group be constituted by *n* genes. When a number of gene sequences greater than two is considered, instead of the linear correlation coefficient, a linear correlation matrix *R*, defined as follows, is considered:

$$R = \begin{vmatrix} 1 & \rho_{12} & \cdots & \rho_{1n} \\ \rho_{21} & 1 & \cdots & \rho_{2n} \\ . & . & \cdots & . \\ \rho_{n1} & \rho_{n2} & \cdots & 1 \end{vmatrix} \qquad (5)$$

$\rho_{ij}$ being the correlation coefficient between the sequences of the gene *i* with the gene *j*. Of course, the correlation coefficient of a gene sequence with itself is 1, that is $\rho_{ij}=1$ for each i=1, ..., *n*.

If for each $i \neq j$ is $\rho_{ij}= 0$, the *n* gene sequences are uncorrelated. In this case the determinant of the matrix *R* is 1, while in general it ranges between 0 and 1.

Considering that $\rho_{ij}=1$ and that $\rho_{ij}=\rho_{ji}$, the number of calculated coefficients is:

$$\binom{n}{2} = \frac{n!}{(n-2)! * 2!} = \frac{n*(n-1)}{2} \qquad (6)$$

Once these values are calculated, the interval from 0 to 1 is divided in sub-intervals, for example in five equal sub-intervals whose amplitude is 0.2 and the number of coefficients comprised in each sub-interval is counted. Moreover, to each sub-interval a correlation value is associated, for example equal to 0.1, 0.3, 0.5, 0.7, 0.9, respectively. If one of the five sub-intervals contains a number of coefficients greater than 50% of the total number of coefficients, the value of the first parameter is the correlation value corresponding to that interval. On the contrary, in the case in which the coefficients are distributed mainly into two intervals, the value of the first parameter is the arithmetical mean of the correlation values of these two intervals.

By assuming for example that $n_1$ coefficients are in the sub-interval to which is associated a correlation value $v_1$, $n_2$ coefficients are in the sub-interval with correlation value $v_2$, and $(n_1+n_2)>50\%$ of the total number of coefficients distributed in the five intervals, the value assigned to the first parameter P1 will be given by:

$$P1 = \frac{(v_1 * n_1 + v_2 * n_2)}{(n_1 + n_2)} \qquad (7)$$

Finally, in the case in which the majority of coefficients is distributed in more than two intervals, the first parameter P1 is the mean value of all the coefficients. The first parameter is calculated considering only the coefficients $\rho_{ij}>0$.

The second parameter P2, relating to the negative linear correlation, is similarly calculated but considering the coefficients $\rho_{ij}<0$ and dividing the interval from -1 to 0 in five equal intervals.

Referring to the calculation of the third parameter P3, the correlation coefficients are calculated by considering the most general form of the correlation coefficient given in eq. (4). Considering that $r_{XY} \neq r_{YX}$, the number of coefficients to be calculated, in the case of a combination with *n* gene sequences, is:

$$n*(n-1) \qquad (8)$$

For calculating *r* it is necessary to know *Ystim,* that is the interpolated value of *Y* by means of the method of minimum squares. The minimum squares parabola interpolating the set of points $(X_i, Y_i)$, with *i*=1, ..., *n* is given by eq. (3):

$$Y=a+bX+cX^2 \tag{3}$$

wherein *a, b* and *c* are determined by solving the following three equations:

$$\begin{cases} \sum Y = aN + b\sum X + c\sum X^2 \\ \sum XY = a\sum X + b\sum X^2 + c\sum X^3 \\ \sum X^2 Y = a\sum X^2 + b\sum X^3 + c\sum X^4 \end{cases} \tag{9}$$

known as the *canonical equations of the minimum squares parabola.*

Given that the values of the constants are known, by substituting them in eq. (3), the value of *Ystim* and thus the value of *r* are calculated.

To the third parameter is attributed the mean value of the so calculated $n*(n-1)$ correlation coefficients.

It should be expected that the combinations CLUSTER-CLUSTER have relatively high correlation values, because the clustering criteria select groups of genes with a high level of correlation among them.

Of course, even CLUSTER-FILTER and FILTER-FILTER correlations may have high correlation values.

In general the correlation parameter provides more complete indications than indications obtained by using clustering criteria.

For a better understanding of this aspect, suppose for example of considering two gene sequences *X* and *Y* constituted by three values of time expression, *X*=[1*;* 5; 7] e *Y*=[10*;* 50; 70]. The relation that ties *X* and *Y* is *Y*=10*X* and so the linear correlation coefficient is 1.

Nevertheless, the clustering criteria do not highlight this kind of relationship. In fact the majority of implemented clustering techniques uses distance metrics. Two gene sequences with very similar values of gene expression are grouped in the same CLUSTER because they identify very close points in the m-dimensional space.

By contrast, in the cited example, even if there is a linear relation between two sequences, they identify distant points of the space and thus, probably, not belonging to the same CLUSTER. The only clustering criterion that make an exception to this rule is the agglomerative method that uses the Pearson's coefficient. In fact, this metric is a measure of similarity and not of distance and does not satisfy metric properties.

**Parameters relating to the pattern of expression**

The last three extracted parameters, P4, P5 and P6, relate to similarity among gene sequences in terms of time-variable or condition-variable pattern of expression. In particular, the sign of the full variation is considered, the type of evolution (increasing or decreasing) and the coincidence of peak variation at the same point of time.

The fourth parameter P4 indicates the percentage of genes that behaves in a similar way from the point of view of the whole variation of the value of gene expression.

For each gene sequence of the examined combination, the variation between the value of final genic expression (that is relating to the last attribute) and the initial one (relating to the first attribute) is calculated, by considering preferably only the absolute value of the variation (independently from the sign). Being the number of gene sequences that have a value of final gene expression greater that the starting known one, the percentage of sequences that have a positive variation is calculated. The fourth parameter is a number comprised between zero and one, indicative of this percentage.

In practice, the closer to 50% the percentage of genes having the same variation is, the closer to 0 the fourth parameter P4 is. The closer to 100% that percentage is, the closer to 1 the parameter P4 is, because the majority of genes of the group behaves in the same way.

In the case in which the percentage is small and close to 0, the value of the parameter is high and is close to 1. This is due to the fact that small percentages of genes having a positive variation, imply high percentages of sequences with negative variation. This parameter is used for identifying groups of genes with a similar behavior from the point of view of the whole variation of the value of gene expression, independently from the sign of the variation. Finally, percentages of 70% or equivalently of 30% are associated to values of the parameter close to 0.5.

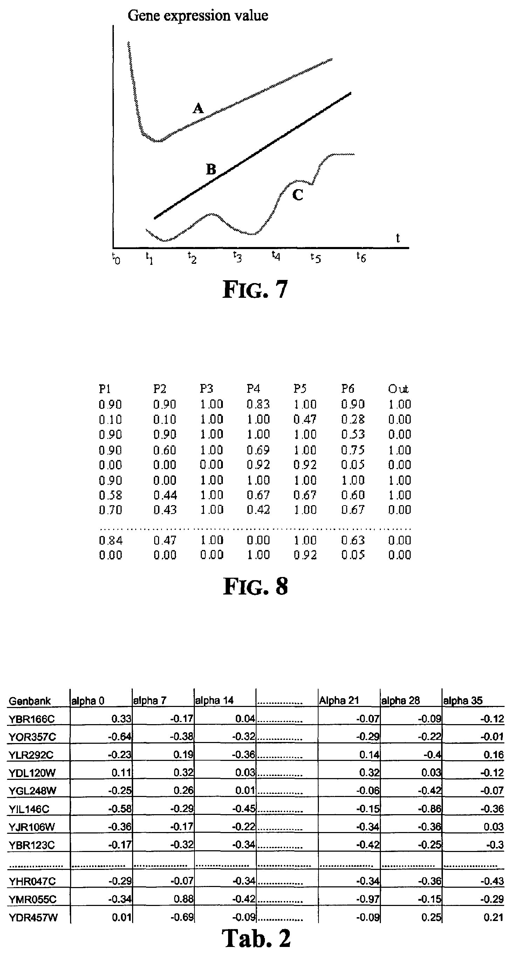

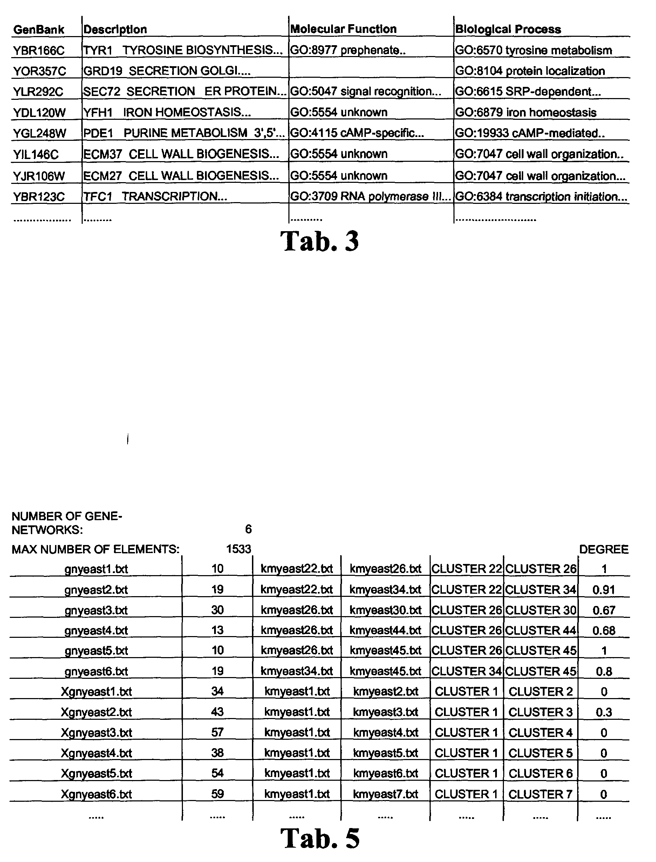

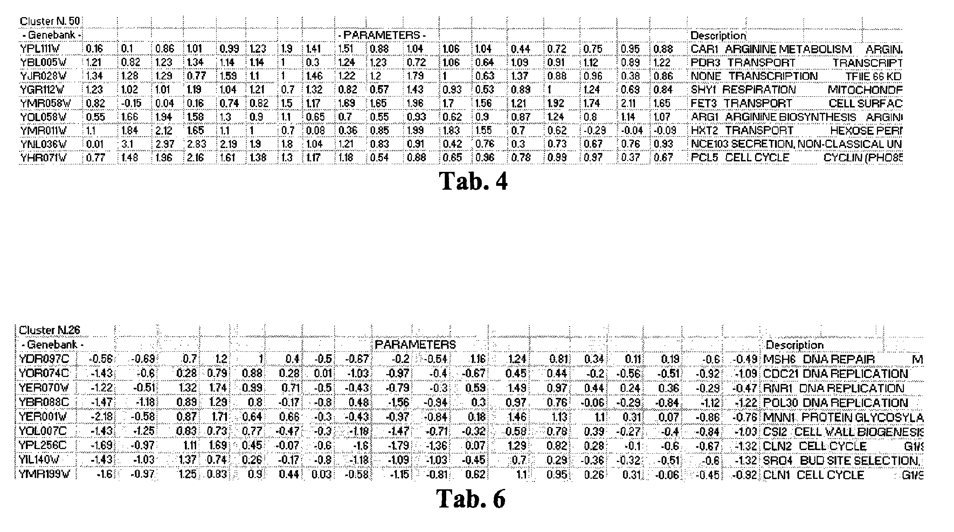

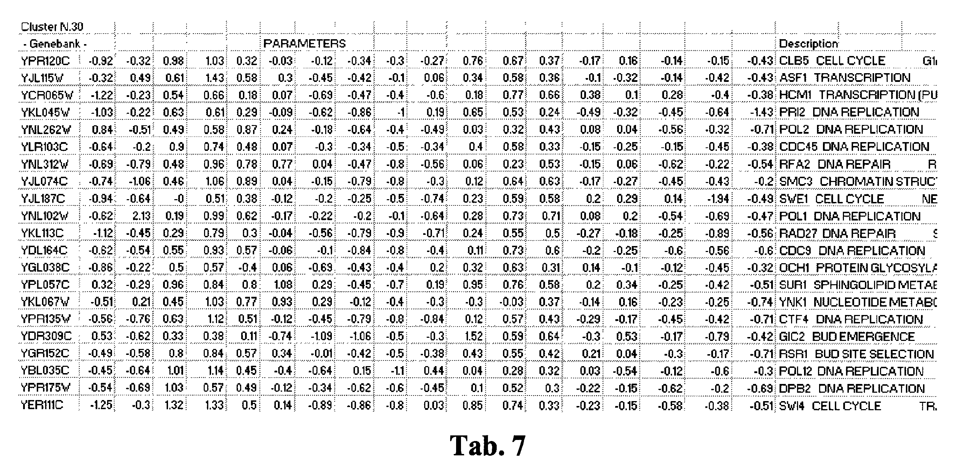

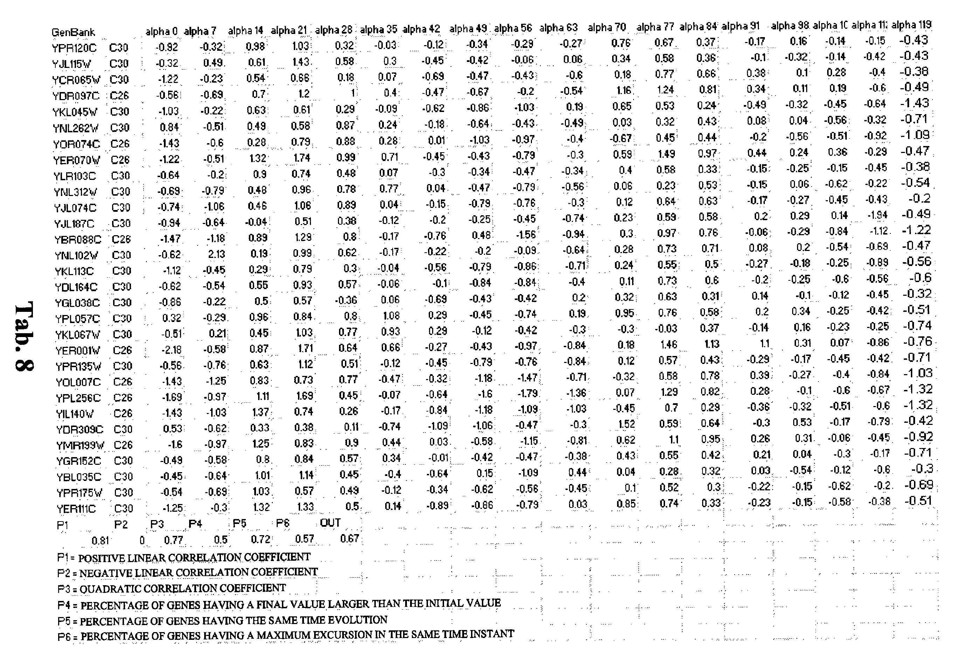

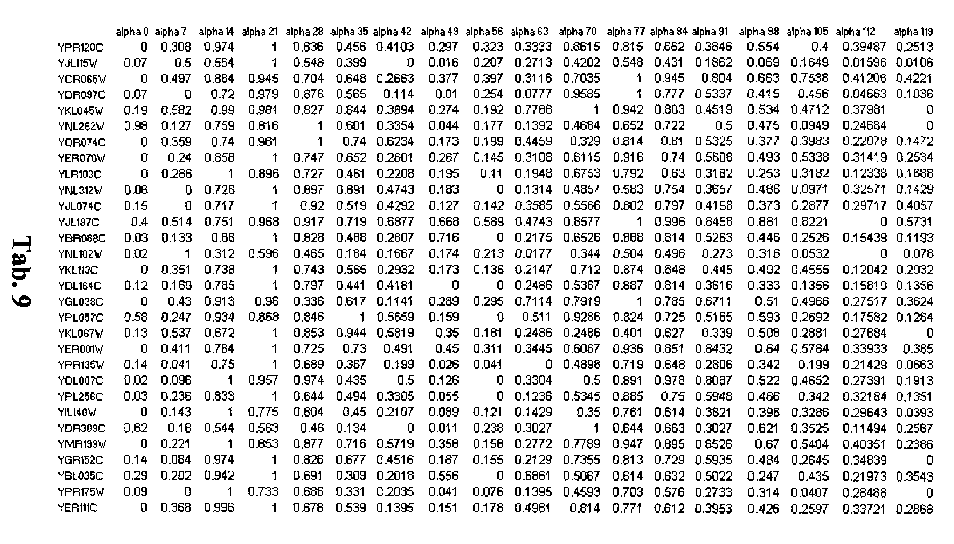

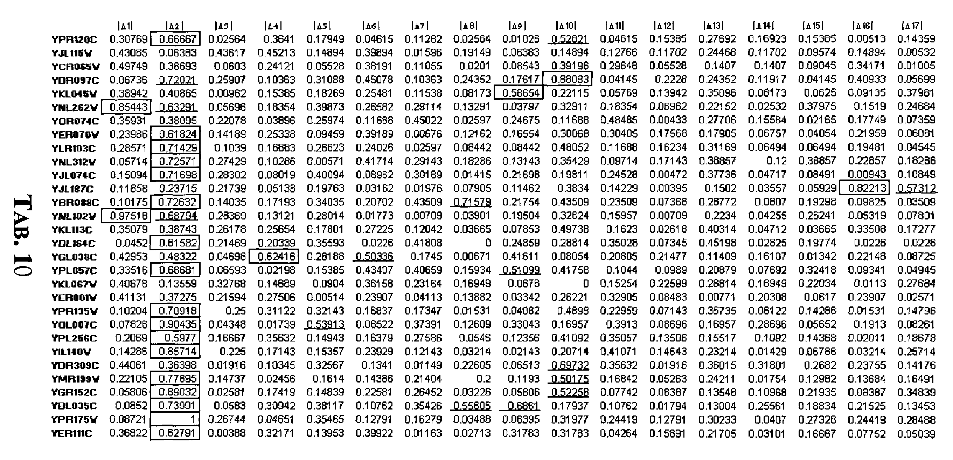

Nevertheless, it must be considered that a gene sequence with a value of final gene expression greater than the initial value not necessarily increases with time, and vice versa a negative variation does not imply that the sequence is decreasing. In order to identify a Gene Network, it is important to identify genes presenting a similar time evolution, whether increasing or decreasing, independently from the values of single attributes. To illustrate this let us consider Figure 7.

The three sample gene sequences A, B, C increase with time, even if their evolution are completely different and the values of gene expression differ among them. Moreover the sequence A, even if it is increasing, has a negative variation between the final and the initial values. This detail is not considered by clustering criteria and it is not highlighted by the four parameters described above.

For this reason a fifth parameter P5 has been introduced for accounting for considering this characteristic. The percentage of genes that present an increasing evolution is calculated for each combination. In function of the obtained percentage, a value ranging from 0 to 1 is assigned to this fifth parameter.

The value assignment is very similar to that illustrated for the fourth parameter: at a very small or very large percentage value corresponds a value of the parameter close to 1, while a value of the parameter close to 0 is associated to a percentage close to 50%.

The sixth parameter P6 refers to gene sequences of the considered group that show a peak variation of the same point of time.

An external cause, such as the administering of a certain substance or the changing of the environmental conditions, such as for example an increase or a decrease of temperature, may cause a strong increase or reduction of the level of genic expression. The coincidence presence of peak variation at the same instant, may lead to the identification of a group of genes that react similarly in presence of a certain external agent.

In consideration of the fact that the values of gene expression are normalized and thus they range between 0 and 1, a threshold value of half the amplitude (0.5) of the normalization interval is considered.

The excursion peak value is calculated for each sequence belonging to the combination to be examined. If no gene of the combination has a peak variation that surpasses the threshold, a null value is assigned to the last parameter P6. If all the genes of the group have a *"peak"* (that is a maximum variation greater that the threshold) occurring at the same point of time, the parameter is 1. In all other cases the parameter has a value equal to the percentage of genes that show a *peak* at the same point of time.

**Intelligent sub-system**

The intelligent sub-system is based on Soft Computing methods, preferably it is a neural fuzzy system whose rules may be either

    1. introduced by the user in form of a programming language using clauses such as IF..THEN; or
    2. generated by means of a neural network with weights and thresholds representing the characteristic parameters.

According to the latter alternative, the sub-system must be trained first (off-line learning) with an appropriate set of data (learning matrix), such as the sample set of data of Figure 8.

While functioning off-line, the output of the Fuzzy system (characteristic value) is compared with a threshold value THRESHOLD. Among the obtained group of genes, the groups associated to a characteristic value greater than the threshold are identified as Gene Network, while the other groups are discarded.

EXAMPLE

For better illustrating the method of this invention, a sample application will now be described. The input data are constituted by levels of genic expression of certain sequences to be examined. Table 2 depicts a portion of the set of data used for the experiment.

In the first column of Table 2 there are the accession numbers of genes, which in this specific case belong to the genome of the yeast *S. cerevisiae* (the first letter of each accession number is Y which stands for "yeast"). For each gene the levels of gene expression in time have been extracted (the name of the experiment is ALPHA and is based on 18 instants) at the end of a cycle of cellular division after synchronization by the *alpha stop factor*. All the measurements were done by taking the value of genic expression at the instant t=0 as reference (second column of the table); the other columns, show the levels of genic expression measured after 7 min, 14 min and so forth.

For each gene, identified by the corresponding accession number, it is possible to gather additional information, such as its description (Description), the functional category (Molecular Function) and the *annotation* (Biological Process) of the specific gene. This information is available in the Saccharomyces Genome Database. An example is shown in Table 3.

**Results obtained with a clustering criterion**

1533 gene sequences relating to the genome of yeast have been considered. Each sequence is constituted by eighteen levels of gene expression, corresponding to the values measured at intervals of 7 minutes, following the ALPHA experiment (instant t=0).

These sequences have been grouped by the algorithm K-means by adopting an initial number of centroids equal to 50 and a random method of generating centroids.

At the end of the clustering process, 50 sub-tables (CLUSTERS) have been obtained, equal to the number of centroids that had been chosen initially when selecting the parameters. This condition indicates that there is not any empty cluster, that eventually would have been discarded at the end of the clustering process.

Table 4 shows the content of the file *kmyeast50. txt* relating to the fiftieth CLUSTER constituted by 9 gene sequences. The file indicates also the accession numbers (GenBank) and the eighteen values of gene expression of the sequences (PARAMETERS) constituting the CLUSTER.

**Filtering phase**

According to this invention it is possible to optionally perform a filtering phase by which it is possible to select several of the considered gene sequences in function of the values of gene expression assumed at different instants. For example it is possible to filter all the sequences whose value of gene expression at the instant *t*=0 is greater than zero or it is possible to consider a filtering criterion relating to more than one parameter at the time. Nevertheless this phase is optional and for sake of simplicity it has been waived for this example.

**Generation of combinations**

During this phase all the combinations CLUSTER-CLUSTER are generated. It is evident that, if the filtering phase had been performed, the combinations FILTER-FILTER and CLUSTER-FILTER would have been generated too.

In this case, considering that the number of combinations is

$$\binom{K}{2} = \frac{K!}{(K-2)! * 2!} = \frac{K * (K-1)}{2}$$

and considering that the number *K* of CLUSTERS generated in the previous phase is 50, 1225 combinations have been obtained.

For each combination the above mentioned six numerical parameters P1, ..., P6 have been calculated.

In function of the calculated parameters, the intelligent sub-system assigned to each combination a characteristic value ranging between 0 and 1.

All the combinations with a characteristic value *(degree of Gene Network),* greater than a pre-established threshold have been identified as possible Gene Networks.

In this example, a threshold value of 0.5 was set and six possible Gene Networks were identified.

The printed file *gnyeast. txt* shown in Table 5 stores information relating to the generated combinations.

In the first column the names of files containing more detailed information on the generated combinations are indicated. The names of the files that start with the letter X refer to the combinations to which the intelligent sub-system assigned a value smaller than 0.5. By contrast, the remaining files refer to the combinations that the system identified as possible Gene Networks, and which in this specific case were 6.

The second column contains the number of gene sequences constituting the examined combination, while the remaining columns indicate the type of combination (example CLUSTER22-CLUSTER26).

The last column represents the value assigned from the previously trained neural fuzzy system. It is evident that the more the assigned value is close to 1, the more likely the examined combination is a Gene Network. Vice versa, the more the output value is close to 0, the more unlikely is the fact that the combination be a Gene Network.

For example looking to the third row, the combination between the CLUSTER26 (C26), constituted by 9 gene sequences, and the CLUSTER30 (C30), constituted by 21 sequences, has a value of 0.67, thus it has been recognized as a possible gene network.

Tables 6 and 7 contain data relating to CLUSTER26 (C26) and CLUSTER30 (C30).

All necessary information relative to the combined set of data is reported in Table 8.

The second column of Table 8 indicates the CLUSTER, to which the gene sequence belongs, whose accession number is written in the first column. Besides the values of gene expression of the sequences that constitute the combination, also the values of the six extracted parameters and the relative output value assigned by the system are shown.

Setting aside the calculation of the first three parameters P1, P2 and P3 relating to the linear and quadratic correlation of the combination, for the sake of clarity, the procedure for calculating the parameters P4, P5 and P6 is described for the combination C26-C30 of Table 8.

**Calculation of P4**

In order to calculate the parameter P4, percentage of genes whose final value is greater than the initial value, for each sequence of the combination the variation $\Delta$ between the value of gene expression corresponding to the last instant *alphaI* 119 and the value of expression corresponding to the first instant *alpha0* must be considered. For the first sequence YPR120C the variation $\Delta$ is:

$$\Delta = -0.43 - (- 0.92) = 0.49 => \Delta > 0$$

This calculation is performed for all the sequences of the combination. The result is that 21 sequences out of 30, that is 70% of the sequences have a positive variation ($\Delta>0$). This percentage, which will be indicated with the variable "PERCENT_VALUE" hereinafter, is transformed in a value comprised between 0 and 1.

The transformation procedure of this variable is:

- When PERCENT_VALUE=50%, P4 is null.
- When 50%<PERCENT_VALUE<70%, P4 is a value ranging from 0 to 0.5 (the greater the percent value, the greater the value of the parameter).
- When 70%<PERCENT_VALUE≤100%, P4 is a value ranging from 0.5 to 1 (the greater the percent value, the greater the parameter).
- When 30%≤PERCENT_VALUE<50%, P4 is a value ranging from 0 to 0.5 (the smaller the percent value, the greater the value of the parameter).
- When 0%<PERCENT_VALUE<30%, P4 is a value ranging from 0.5 to 1 (the smaller the percent value, the greater the value of the parameter).

In practice, the closer to 50% is the percentage of genes having the same variation, the closer to 0 is the value of the fourth parameter P4. The closer to 100% is the PERCENT_VALUE, the closer to 1 is the parameter P4, because the majority of genes of the group has a similar behavior.

In the case in which the percentage is small and close to 0, the value of the parameter P4 is great and is close to 1. This is due to the fact that small percentages of genes having a positive variation ($\Delta>0$) imply high percentages of sequences with negative variation ($\Delta<0$). This parameter allows identification of groups of genes with a similar behavior from the point of view of the global variation of the value of gene expression, independently from the sign of the variation.

In the considered example, given that the percent value is 70%, P4 has a value equal to 0.5 (see Table 8).

**Calculation of P5**

In order to calculate P5, which is the percentage of genes with the same time evolution, for each sequence of the combination it must be verified whether the pattern of gene expression is increasing or decreasing with time.

Given that the values of gene expression are sampled, the variations $\Delta i$ between the gene expression value corresponding to the $(i)^{th}$ instant and the value of the gene expression corresponding to the $(i-1)^{th}$ for $i$=1, 2, ..., n being $n$ the number of experiments, must be calculated. In this specific case $n$=18 and for each sequence $n$-1 (17) values are calculateFor example, for the first sequence YPR120C the values of the variations $\Delta i$ are:

$$\Delta = alpha7 - alpha0 = -0.32 - ( 0. 92) = 0.6 > 0$$

$$\Delta2 = alpha14 - alpha7 = 0.98 - (-0.32) = 1.3 > 0$$

$$\Delta3 = alpha21 - alpha14 = 1.03 - 0.98 = 0.05 > 0$$

$$\Delta 4 = alpha28\text{-}alpha21 = 0.32\ \text{-}1.03 < 0$$

$$\Delta 5 = alpha35\text{-}alpha28 = \text{-}0.03\text{-}0.32 < 0$$

$$\Delta 6 = alpha42\text{-}alpha35 = \text{-}0.12\text{+}0.03 < 0$$

$$\Delta 7 = alpha49\text{-}alpha42 = \text{-}0.34\text{+}0.12 < 0$$

$$\Delta 8 = alpha56\text{-}alpha49 = \text{-}0.29\text{+}0.34 > 0$$

$$\Delta 9 = alpha63\text{-}alpha56 = \text{-}0.27\text{+}0.29 > 0$$

$$\Delta 10 = alpha70\text{-}alpha63 = 0.76\text{+}0.27 > 0$$

$$\Delta 11 = alpha77\text{-}alpha70 = 0.67\text{-}0.76 < 0$$

$$\Delta 12 = alpha84\text{-}alpha77 = 0.37\text{-}0.67 < 0$$

$$\Delta 13 = alpha91\text{-}alpha84 = \text{-}0.17\text{-}0.37 < 0$$

$$\Delta 14 = alpha98\text{-}alpha91 = 0.16\text{+}0.17 > 0$$

$$\Delta 15 = alpha105\text{-}alpha98 = \text{-}0.14\text{-}0.16 < 0$$

$$\Delta 16 = alpha112\text{-}alpha105 = \text{-}0.15\ \text{+}0.14 < 0$$

$$\Delta 17 = alpha119\text{-}alpha112 = \text{-}0.43\ \text{+}0.15 < 0$$

If the number of positive variations $\Delta i$ is greater than the number of negative variations $\Delta i$, the time evolution of the sequence is globally increasing, vice versa it is globally decreasing.

For the sequence YPR120C, the number of positive variations $\Delta i$ is 7, while the number of negative variations $\Delta i$ is 10. Given that the number of positive variations $\Delta i$ is smaller than the number of negative variations $\Delta i$, the sequence is considered having a decreasing time evolution.

The same calculation is repeated for each of the remaining 29 sequences of the combination illustrated in Table 8, obtaining that a certain percentage of sequences (indicated hereinafter with the variable "PERCENT") has a globally increasing time evolution. To this percent value a parameter P5, whose value ranges between 0 and 1, is associated with a procedure similar to that described for the parameter P4.

In the considered case, 5 sequences out of 30, that is 16.7% has a globally increasing time evolution, thus P5 is large. In fact 83.3% of sequences has a decreasing evolution and thus a higher percentage of sequences has similar time evolutions.

It is worth remarking that the value of parameter P5 does not depend on the fact that the global evolution of the majority of genes is increasing or decreasing, but on how many genes of the combination have the same evolution. In the considered case, the parameter (P5) is close to 1.

A possible procedure for evaluating P5 is described in detail hereinafter.

Let us define three threshold values
THRESHOLD1=0.3; THRESHOLD2=1-THRESHOLD1=0.7; THRESHOLD3=0.5;
and in function of the thresholds the following values are calculated:

$$\text{VALUE 1}=((\text{THRESHOLD2}-\text{THRESHOLD3})/(1-\text{THRESHOLD3}))=0.4;$$

$$\text{THRESHOLD2}=((2*\text{THRESHOLD2}-1+\text{THRESHOLD3})/(2*\text{THRESHOLD3}))=0.9;$$

- If THRESHOLD2<=PERCENT<= 1, P5=((PERCENT-THRESHOLD1)/(1-VALUE1));
- If 0<=PERCENT<=THRESHOLD1, P5=(((1-PERCENT)-VALUE1)/(1-VALUE1));
- If 0.5<=PERCENT<THRESHOLD2, P5=((PERCENT-0.5)/(VALUE2-0.5));
- If THRESHOLD1<PERCENT<0.5, P5=(((1-PERCENT)-0.5)/(VALUE2-0.5)).

A percentage of 50% corresponds to a null value of P5 because, as previously stated, in this case there is not any prevalent (increasing or decreasing) time evolution of the sequences that constitute the combination.
In the proposed example (Table 8) the value of P5 is given by:

$$P5=(((1-\text{PERCENT})-\text{VALUE 1})/(1-\text{VALUE1}))=(1-0.167-0.4)/0.6=0.72$$

**Calculation of P6**

In order to calculate P6, percentage of genes with a peak occurring at the same instant, for each sequence of the combination it must be verified whether the absolute value of the variation $\Delta i$ exceeds a certain threshold value. Given that the values relative to each experiment have been normalized between 0 and 1, a value of 0.5, which is one half of the normalization interval, was chosen as threshold value. Table 9 contains the gene expression values normalized between 0 and 1 of the sequences constituting the combination C26-C30.
For sequence YPR120C is:

$$|\Delta 1|=alpha7-alpha0| = 0.307$$

$$|\Delta 2| =|alpha14-alpha7| = 0.666$$

$$................................................$$

$$|\Delta 17|=|alpha19-alpha112| = 0.14359$$

Repeating these calculations for all the sequences, the results reported in Table 10 are obtained.
The values of $|\Delta|$ that surpass the threshold are underlined. For each sequence the maximum value of $|\Delta|$ that surpasses the threshold is the *peak* (maximum value) and it is highlighted with a continuous line perimeter. For example, in correspondence of the first sequence YPR120C there are only two values greater than the threshold, $|\Delta 2|$ = 0.66 and $|\Delta 10|$ = 0.52; the peak in this case is $|\Delta 2|$.
It must be noticed that not all sequences necessarily show a peak. In the proposed example the sequences YJL115W, YCR065W, YOR074C, YKL113C, YKL076W, YER00 Wand YDR309C do not show a peak.
For calculating the sixth parameter P6, the maximum number of peaks occurring at the same instant must be considered. In this example, the maximum number of peaks is 17 and they are in the column of $|\Delta 2|$. In particular, 56.7% of sequences (17 sequences out of 30) of the combination have a coincident peak and thus in this case P6 is equal to 0.57, as shown in Table 8.

**REFERENCES**

[1] Luke Alphey, *DNA Sequencing:from experimental methods to bioinformatics,* BIOS Scientific Publishers, 1997.

[2] G. Lewin, *Gene IV,* 1998.

[3] D.L. Kirk, *Biologia Oggi,* Piccinini editore, Padova.

[4] Chieffi, Dolfini, Malcovati, Pierantoni, Tenchini, *Biologia e Genetica,* EdiSES.

[5] M.L.M. Anderson, *Nucleic Acid Hybridization,* BIOS scientific Publishers, 1999.

[6] M. Schena, *DNA Microarray: A Practical Approach,* Oxford University Press 1999.

[7] Patrik D'Haeseller, *Reconstructing Gene Networks from Large Scale Gene Expression Data,* The University of New Mexico, December 2000.

[8] Pierre Bladi, Soren Brunak, *Bioinformatics. The Machine Learning Approach,* MIT Press 1998.

[9] Primer on Molecular Genetic, *DOE Human Genome Program, 1992.*

[10] M.B.Eisen, P.T.Spellman, Patrick O.Brown, D.Botstein, *Cluster Analysis and display of genome-wide expression patterns,* Vol. 95, pp. 14863-14868, December 98, Genetics.

[11] P.D'Haeseleer, S.Liang, R. Somogyi, *Genetic Network inference: from co-expression clustering to reverse engineering,* Vol. 16 no.8 2000, pages 707-726.

[12] S.Huang, *Gene expression profiling, genetic networks and cellular state*s: *an integrating concept for tumorigenesis and drug discovery,* 1 July 1999.

[13] P.Smolen, D. A. Baxter, J.H. Byrne, *Mathematical Modeling of Gene Networks,* Neuron, Vol.26, 567-580, June 2000.

[14] Frank Höppner, Frank Klawonn, *"Fuzzy Cluster Analysis: Methods for Classification Data Analysis and Image Recognition",* first edition 1999.

[15] Michele Scardi, *"Tecniche di Analisi dei Dati in Ecologia",* Version 1.2a, April 1998.

[16] Daniel Boley, Vivian Borst, *"Unsupervised Clustering: A Fast Scalable Method for Large Datasets",* Department of Computer Science and Engineering.

[17] D.L. Boley, Principal Direction Divisive Partitioning, *Data Mining and Knowledge Discovery,* 1999, Department of Computer Science and Engineering.

[18] Kohonen T. *Self-Organization and Associative Memory,* Primavera 1984.

[19] Flexer A. *On the Use of Self Organizing Maps for Clustering and Visualization.*

[20] R.J. Hataway, J.C.Bezdek, Y.Hu, *Generalized Fuzzy c-Means Clustering Strategies Using Lp Norm Distances,* IEEE Transactions on fuzzy system, Vol. 8, No 5, October 2000.

[21] R.Krishapuram, J.Keller, *A Possibilistic Approach to Clustering,* IEEE Transactions on fuzzy system, Vol. 1, No 2, May 1993.

[22] Lotfi A. Zadeh, *"Fuzzy logic, Neural Networks, and SoftComputing",* Comm. of the ACM, March 1994, vol 37, No.3.

[23] XL. Daboni, *Calcolo delle probabilità ed elementi di Statistica.*

**Claims**

1. A method of analyzing a table of data relating to gene expression that varies with time and/or with the changing of environmental conditions, to identify groups of co-regulated and co-expressed genes, comprising

   defining a clustering criterion of the data of said table;
   for said clustering criterion, identifying groups of genes in sub-tables (CLUSTERS) that satisfy the defined clustering criterion;
   generating combinations of pairs of said sub-tables;
   calculating characteristic parameters of data associated to genes of a same combination;
   generating a characteristic value defined in function of said parameters for each of said groups of genes by a decision algorithm based on soft computing techniques;
   identifying the combinations whose characteristic value is greater than a certain pre-established threshold as *'Gene Networks'* and discarding groups of genes whose characteristic value is smaller than said threshold.

2. The method of claim 1, further comprising the operations of

   defining a respective set of logic filtering criteria of data of said table;
   for each logic filtering criterion, determining a corresponding filtered sub-table (FILTER) containing data of genes whose expression values satisfy said logic filtering criterion;
   generating combinations of pairs of sub-tables obtained with said logic filtering criteria and clustering criterion.

3. The method of claim 1, wherein said decision algorithm is a fuzzy logic algorithm whose antecedents and consequents are defined in function of said parameters.

4. The method of claim 1, wherein said parameters are chosen in the set constituted by numerical parameters tied to gene expression levels, parameters that have a semantic biological meaning, and by mixed parameters that express at the same time numerical relations and a semantic meaning.

5. The method of claim 1, wherein said parameters and correlation indexes are chosen in the set constituted by:

   absolute values of linear correlation coefficients among data associated to pairs of genes;
   absolute values of quadratic correlation coefficients among data associated to pairs of genes;
   percentage of genes of the combination whose final value of gene expression is greater than the respective value of initial gene expression;
   percentage of genes of the combination whose final value of gene expression is smaller than the respective value of initial gene expression;
   percentage of genes whose values of gene expression have a same increasing or decreasing time evolution;
   percentage of genes that have a maximum value of gene expression in a same condition;
   percentage of genes that have ontologies in common;
   percentage of genes that have functional domains in common.

6. The method of claim 1, further comprising discarding combinations among sub-tables constituted by a number of genes smaller than a certain pre-established number, considering only once the genes that are comprised in both combined sub-tables.

7. The method of claim 1, wherein said clustering criteria are based on algorithms chosen in the set constituted by agglomerative hierarchic algorithms, non hierarchic Kmeans algorithms, hierarchic sequential Kmeans, non hierarchic SOM and not exclusive Fuzzy Clustering.

8. The method of claim 5, comprising

   calculating the correlation coefficients of all pairs of gene sequences of the combination;
   subdividing the interval from 0 to 1 in five sub-intervals of equal length and assigning to each of said sub-intervals a respective quantized value of correlation $(v_i)$;

calculating the percentage of correlation coefficients belonging to each sub-interval;
defining for each combination an overall coefficient of linear correlation obtained as arithmetic mean of the quantized values associated to the sub-intervals containing a number of coefficient greater than 50%.

9. The method of claim 5, comprising

calculating coefficients of quadratic correlation of all pairs of gene sequences of a same combination;
defining for each combination a global coefficient of quadratic correlation obtained as arithmetic mean of said correlation values.

10. The method of claim 5, comprising

calculating the percentage of sequence of the combination with a final value of gene expression greater than the initial value of gene expression;
defining a coefficient of global variation of the value of gene expression, comprised between 0 and 1, corresponding to said percentage.

11. The method of claim 5, comprising

calculating the percentage of sequences of the combination with an increasing time evolution;
defining a coefficient relative to the time evolution of the gene expression comprised between 0 and 1 corresponding to said percentage.

12. The method of claim 5, comprising

calculating the percentage of sequences of the combination with a value of gene expression greater than a pre-established threshold in correspondence of a same instant;
defining a coefficient of presence of maximum excursion of the level of gene expression in correspondence of the same instant, comprised between 0 and 1, corresponding to said percentage.

13. An identification system of groups of co-expressed and co-regulated genes, according to the method of claim 1, comprising

a pre-processing sub-system input with data of a table relative to gene expressions variable with time or relating to different conditions, generating sub-tables (CLUSTERS) of data in groups of genes that satisfy a pre-established clustering criterion;
a processing sub-system of data of said sub-tables (CLUSTERS), generating signals representing characteristic parameters of data associated to genes of a same combination of pairs of said sub-tables;
an intelligent sub-system input with said signals representative of characteristic parameters and outputting data of groups of genes identified as *'Gene Networks'*.

14. The system of claim 13, wherein said intelligent sub-system is an neural fuzzy sub-system trained off-line.

| ORF | 0 Minutes | 30 Minutes | 1 Hour | 2 Hours |
|---|---|---|---|---|
| YAL001C | 1 | 1.3 | 2.4 | 5.8 |
| YAL002W | 0.9 | 0.8 | 0.7 | 0.5 |
| YAL003W | 0.8 | 2.1 | 4.2 | 10.1 |
| YAL005C | 1.1 | 1.3 | 0.8 | |
| YAL010C | 1.2 | 1 | 1.1 | 4.5 |

## TAB. 1

## FIG. 1

## FIG. 2

NCBI Reference Sequences (RefSeq)        ?

**Category:** PROVISIONAL

| | | |
|---|---|---|
| **mRNA:** | NM_017106 | |
| **Protein:** | NP_058802   chloride channel 5 | **BL** |
| **Domains:** | CBS domain | score: 103 |
| | Biotin synthase | score: 86 |
| | Voltage gated chloride channels | score: 858 |
| | Domain in cystathionine beta-synthase and other proteins. | score: 108 |
| **GenBank Source:** | D50497 | |

GenBank Sequences        ?

| Nucleotide | Type | Protein | |
|---|---|---|---|
| D50497 | m | BAA09091 | **BL** |
| Z56277 | m | CAA91216 | **BL** |

Function     Submit GeneRI       ?

**Gene Ontology™:**

| Term | Evidence | Source | Pub |
|---|---|---|---|
| • membrane | IEA | RGD | |
| • chloride transport | IEA | RGD | |

## FIG. 3

## FIG. 4

FIG. 5

FIG. 6

Gene expression value

**FIG. 7**

| P1 | P2 | P3 | P4 | P5 | P6 | Out |
|------|------|------|------|------|------|------|
| 0.90 | 0.90 | 1.00 | 0.83 | 1.00 | 0.90 | 1.00 |
| 0.10 | 0.10 | 1.00 | 1.00 | 0.47 | 0.28 | 0.00 |
| 0.90 | 0.90 | 1.00 | 1.00 | 1.00 | 0.53 | 0.00 |
| 0.90 | 0.60 | 1.00 | 0.69 | 1.00 | 0.75 | 1.00 |
| 0.00 | 0.00 | 0.00 | 0.92 | 0.92 | 0.05 | 0.00 |
| 0.90 | 0.00 | 1.00 | 1.00 | 1.00 | 1.00 | 1.00 |
| 0.58 | 0.44 | 1.00 | 0.67 | 0.67 | 0.60 | 1.00 |
| 0.70 | 0.43 | 1.00 | 0.42 | 1.00 | 0.67 | 0.00 |
| ... | ... | ... | ... | ... | ... | ... |
| 0.84 | 0.47 | 1.00 | 0.00 | 1.00 | 0.63 | 0.00 |
| 0.00 | 0.00 | 0.00 | 1.00 | 0.92 | 0.05 | 0.00 |

**FIG. 8**

| Genbank | alpha 0 | alpha 7 | alpha 14 | ........... | Alpha 21 | alpha 28 | alpha 35 |
|---------|---------|---------|----------|-------------|----------|----------|----------|
| YBR166C | 0.33 | -0.17 | 0.04 | ........... | -0.07 | -0.09 | -0.12 |
| YOR357C | -0.64 | -0.38 | -0.32 | ........... | -0.29 | -0.22 | -0.01 |
| YLR292C | -0.23 | 0.19 | -0.36 | ........... | 0.14 | -0.4 | 0.16 |
| YDL120W | 0.11 | 0.32 | 0.03 | ........... | 0.32 | 0.03 | -0.12 |
| YGL248W | -0.25 | 0.26 | 0.01 | ........... | -0.06 | -0.42 | -0.07 |
| YIL146C | -0.58 | -0.29 | -0.45 | ........... | -0.15 | -0.86 | -0.36 |
| YJR106W | -0.36 | -0.17 | -0.22 | ........... | -0.34 | -0.36 | 0.03 |
| YBR123C | -0.17 | -0.32 | -0.34 | ........... | -0.42 | -0.25 | -0.3 |
| ........... | ........... | ........... | ........... | ........... | ........... | ........... | ........... |
| YHR047C | -0.29 | -0.07 | -0.34 | ........... | -0.34 | -0.36 | -0.43 |
| YMR055C | -0.34 | 0.88 | -0.42 | ........... | -0.97 | -0.15 | -0.29 |
| YDR457W | 0.01 | -0.69 | -0.09 | ........... | -0.09 | 0.25 | 0.21 |

**Tab. 2**

| GenBank | Description | Molecular Function | Biological Process |
|---|---|---|---|
| YBR166C | TYR1 TYROSINE BIOSYNTHESIS... | GO:8977 prephenate.. | GO:6570 tyrosine metabolism |
| YOR357C | GRD19 SECRETION GOLGI.... | | GO:8104 protein localization |
| YLR292C | SEC72 SECRETION ER PROTEIN... | GO:5047 signal recognition... | GO:6615 SRP-dependent... |
| YDL120W | YFH1 IRON HOMEOSTASIS... | GO:5554 unknown | GO:6879 iron homeostasis |
| YGL248W | PDE1 PURINE METABOLISM 3',5'... | GO:4115 cAMP-specific... | GO:19933 cAMP-mediated.. |
| YIL146C | ECM37 CELL WALL BIOGENESIS... | GO:5554 unknown | GO:7047 cell wall organization.. |
| YJR106W | ECM27 CELL WALL BIOGENESIS... | GO:5554 unknown | GO:7047 cell wall organization... |
| YBR123C | TFC1 TRANSCRIPTION... | GO:3709 RNA polymerase III... | GO:6384 transcription initiation... |
| .................... | .......... | .......... | ......................... |

# Tab. 3

NUMBER OF GENE-NETWORKS: 6

MAX NUMBER OF ELEMENTS: 1533

| | | | | | | DEGREE |
|---|---|---|---|---|---|---|
| gnyeast1.txt | 10 | kmyeast22.txt | kmyeast26.txt | CLUSTER 22 | CLUSTER 26 | 1 |
| gnyeast2.txt | 19 | kmyeast22.txt | kmyeast34.txt | CLUSTER 22 | CLUSTER 34 | 0.91 |
| gnyeast3.txt | 30 | kmyeast26.txt | kmyeast30.txt | CLUSTER 26 | CLUSTER 30 | 0.67 |
| gnyeast4.txt | 13 | kmyeast26.txt | kmyeast44.txt | CLUSTER 26 | CLUSTER 44 | 0.68 |
| gnyeast5.txt | 10 | kmyeast26.txt | kmyeast45.txt | CLUSTER 26 | CLUSTER 45 | 1 |
| gnyeast6.txt | 19 | kmyeast34.txt | kmyeast45.txt | CLUSTER 34 | CLUSTER 45 | 0.8 |
| Xgnyeast1.txt | 34 | kmyeast1.txt | kmyeast2.txt | CLUSTER 1 | CLUSTER 2 | 0 |
| Xgnyeast2.txt | 43 | kmyeast1.txt | kmyeast3.txt | CLUSTER 1 | CLUSTER 3 | 0.3 |
| Xgnyeast3.txt | 57 | kmyeast1.txt | kmyeast4.txt | CLUSTER 1 | CLUSTER 4 | 0 |
| Xgnyeast4.txt | 38 | kmyeast1.txt | kmyeast5.txt | CLUSTER 1 | CLUSTER 5 | 0 |
| Xgnyeast5.txt | 54 | kmyeast1.txt | kmyeast6.txt | CLUSTER 1 | CLUSTER 6 | 0 |
| Xgnyeast6.txt | 59 | kmyeast1.txt | kmyeast7.txt | CLUSTER 1 | CLUSTER 7 | 0 |
| ..... | ..... | ..... | ..... | ..... | ..... | ..... |

# Tab. 5

**Cluster N. 50**

| - Genebank - | | | | | | | | - PARAMETERS - | | | | | | | | | | Description |
|---|---|---|---|---|---|---|---|---|---|---|---|---|---|---|---|---|---|---|
| YPL111W | 0.16 | 0.1 | 0.86 | 1.01 | 0.99 | 1.23 | 1.9 | 1.41 | 1.51 | 0.88 | 1.04 | 1.06 | 1.04 | 0.44 | 0.72 | 0.75 | 0.95 | 0.88 | CAR1 ARGININE METABOLISM  ARGIN. |
| YBL005W | 1.21 | 0.82 | 1.23 | 1.34 | 1.14 | 1.14 | 1 | 0.3 | 1.24 | 1.23 | 0.72 | 1.06 | 0.64 | 1.09 | 0.91 | 1.12 | 0.89 | 1.22 | PDR3 TRANSPORT  TRANSCRIPT |
| YJR028W | 1.34 | 1.28 | 1.29 | 0.77 | 1.59 | 1.1 | 1 | 1.46 | 1.22 | 1.2 | 1.79 | 1 | 0.63 | 1.37 | 0.88 | 0.96 | 0.38 | 0.86 | NONE TRANSCRIPTION  TFIIE 66 KD |
| YGR112W | 1.23 | 1.02 | 1.01 | 1.19 | 1.04 | 1.21 | 0.7 | 1.32 | 0.82 | 0.57 | 1.43 | 0.93 | 0.53 | 0.89 | 1 | 1.24 | 0.69 | 0.84 | SHY1 RESPIRATION  MITOCHONDF |
| YMR058W | 0.82 | -0.15 | 0.04 | 0.16 | 0.74 | 0.82 | 1.5 | 1.17 | 1.69 | 1.65 | 1.96 | 1.7 | 1.56 | 1.21 | 1.92 | 1.74 | 2.11 | 1.65 | FET3 TRANSPORT  CELL SURFAC |
| YOL058W | 0.55 | 1.66 | 1.94 | 1.58 | 1.3 | 0.9 | 1.1 | 0.65 | 0.7 | 0.55 | 0.93 | 0.62 | 0.9 | 0.87 | 1.24 | 0.8 | 1.14 | 1.07 | ARG1 ARGININE BIOSYNTHESIS  ARGINI |
| YMR011W | 1.1 | 1.84 | 2.12 | 1.65 | 1.1 | 1 | 0.7 | 0.08 | 0.36 | 0.85 | 1.99 | 1.83 | 1.55 | 0.7 | 0.62 | -0.29 | -0.04 | -0.09 | HXT2 TRANSPORT  HEXOSE PERI |
| YNL036W | 0.01 | 3.1 | 2.97 | 2.83 | 2.19 | 1.9 | 1.8 | 1.04 | 1.21 | 0.83 | 0.91 | 0.42 | 0.76 | 0.3 | 0.73 | 0.67 | 0.76 | 0.93 | NCE103 SECRETION, NON-CLASSICAL UN |
| YHR071W | 0.77 | 1.48 | 1.96 | 2.16 | 1.61 | 1.38 | 1.3 | 1.17 | 1.16 | 0.54 | 0.88 | 0.65 | 0.96 | 0.78 | 0.99 | 0.97 | 0.37 | 0.67 | PCL5 CELL CYCLE  CYCLIN (PHO85 |

**Tab. 4**

**Cluster N.26**

| - Genebank - | | | | | | | | PARAMETERS | | | | | | | | | | Description |
|---|---|---|---|---|---|---|---|---|---|---|---|---|---|---|---|---|---|---|
| YDR097C | -0.56 | -0.69 | 0.7 | 1.2 | 1 | 0.4 | -0.5 | -0.67 | -0.2 | -0.54 | 1.16 | 1.24 | 0.81 | 0.34 | 0.11 | 0.19 | -0.6 | -0.49 | MSH6 DNA REPAIR  M |
| YOR074C | -1.43 | -0.6 | 0.28 | 0.79 | 0.88 | 0.28 | 0.01 | -1.03 | -0.97 | -0.4 | -0.67 | 0.45 | 0.44 | -0.2 | -0.56 | -0.51 | -0.92 | -1.09 | CDC21 DNA REPLICATION |
| YER070W | -1.22 | -0.51 | 1.32 | 1.74 | 0.99 | 0.71 | -0.5 | -0.43 | -0.79 | -0.3 | 0.59 | 1.49 | 0.97 | 0.44 | 0.24 | 0.36 | -0.29 | -0.47 | RNR1 DNA REPLICATION |
| YBR088C | -1.47 | -1.18 | 0.89 | 1.29 | 0.8 | -0.17 | -0.8 | 0.48 | -1.56 | -0.94 | 0.3 | 0.97 | 0.76 | -0.06 | -0.29 | -0.84 | -1.12 | -1.22 | POL30 DNA REPLICATION |
| YER001W | -2.18 | -0.58 | 0.87 | 1.71 | 0.64 | 0.66 | -0.3 | -0.43 | -0.97 | -0.84 | 0.18 | 1.46 | 1.13 | 1.1 | 0.31 | 0.07 | -0.86 | -0.76 | MNN1 PROTEIN GLYCOSYLA |
| YOL007C | -1.43 | -1.25 | 0.83 | 0.73 | 0.77 | -0.47 | -0.3 | -1.18 | -1.47 | -0.71 | -0.32 | 0.58 | 0.78 | 0.39 | -0.27 | -0.4 | -0.84 | -1.03 | CSI2 CELL WALL BIOGENESIS |
| YPL256C | -1.69 | -0.97 | 1.11 | 1.69 | 0.45 | -0.07 | -0.6 | -1.6 | -1.79 | -1.36 | 0.07 | 1.29 | 0.82 | 0.28 | -0.1 | -0.6 | -0.67 | -1.32 | CLN2 CELL CYCLE  G1/S |
| YIL140W | -1.43 | -1.03 | 1.37 | 0.74 | 0.26 | -0.17 | -0.8 | -1.18 | -1.09 | -1.03 | -0.45 | 0.7 | 0.29 | -0.36 | -0.32 | -0.51 | -0.6 | -1.32 | SRO4 BUD SITE SELECTION, |
| YMR199W | -1.6 | -0.97 | 1.25 | 0.83 | 0.9 | 0.44 | 0.03 | -0.58 | -1.15 | -0.81 | 0.62 | 1.1 | 0.95 | 0.26 | 0.31 | -0.06 | -0.45 | -0.92 | CLN1 CELL CYCLE  G1/S |

**Tab. 6**

Cluster N.30

| - Genebank - | PARAMETERS | | | | | | | | | | | | | | | | | | Description |
|---|---|---|---|---|---|---|---|---|---|---|---|---|---|---|---|---|---|---|---|
| YPR120C | -0.92 | -0.32 | 0.98 | 1.03 | 0.32 | -0.03 | -0.12 | -0.34 | -0.3 | -0.27 | 0.76 | 0.67 | 0.37 | -0.17 | 0.16 | -0.14 | -0.15 | -0.43 | CLB5 CELL CYCLE G1 |
| YJL115W | -0.32 | 0.49 | 0.61 | 1.43 | 0.58 | 0.3 | -0.45 | -0.42 | -0.1 | 0.06 | 0.34 | 0.58 | 0.36 | -0.1 | -0.32 | -0.14 | -0.42 | -0.43 | ASF1 TRANSCRIPTION |
| YCR065W | -1.22 | -0.23 | 0.54 | 0.66 | 0.18 | 0.07 | -0.69 | -0.47 | -0.4 | -0.6 | 0.18 | 0.77 | 0.66 | 0.38 | 0.1 | 0.28 | -0.4 | -0.38 | HCM1 TRANSCRIPTION (PU |
| YKL045W | -1.03 | -0.22 | 0.63 | 0.61 | 0.29 | -0.09 | -0.62 | -0.86 | -1 | 0.19 | 0.65 | 0.53 | 0.24 | -0.49 | -0.32 | -0.45 | -0.64 | -1.43 | PRI2 DNA REPLICATION |
| YNL262W | 0.84 | -0.51 | 0.49 | 0.58 | 0.87 | 0.24 | -0.18 | -0.64 | -0.4 | -0.49 | 0.03 | 0.32 | 0.43 | 0.08 | 0.04 | -0.56 | -0.32 | -0.71 | POL2 DNA REPLICATION |
| YLR103C | -0.64 | -0.2 | 0.9 | 0.74 | 0.48 | 0.07 | -0.3 | -0.34 | -0.5 | -0.34 | 0.4 | 0.58 | 0.33 | -0.15 | -0.25 | -0.15 | -0.45 | -0.38 | CDC45 DNA REPLICATION |
| YNL312W | -0.69 | -0.79 | 0.48 | 0.96 | 0.78 | 0.77 | 0.04 | -0.47 | -0.8 | -0.56 | 0.06 | 0.23 | 0.53 | -0.15 | 0.06 | -0.62 | -0.22 | -0.54 | RFA2 DNA REPAIR R |
| YJL074C | -0.74 | -1.06 | 0.46 | 1.06 | 0.89 | 0.04 | -0.15 | -0.79 | -0.8 | -0.3 | 0.12 | 0.64 | 0.63 | -0.17 | -0.27 | -0.45 | -0.43 | -0.2 | SMC3 CHROMATIN STRUC |
| YJL187C | -0.94 | -0.64 | -0 | 0.51 | 0.38 | -0.12 | -0.2 | -0.25 | -0.5 | -0.74 | 0.23 | 0.59 | 0.58 | 0.2 | 0.29 | 0.14 | -1.94 | -0.49 | SWE1 CELL CYCLE NE |
| YNL102W | -0.62 | 2.13 | 0.19 | 0.99 | 0.62 | -0.17 | -0.22 | -0.2 | -0.1 | -0.64 | 0.28 | 0.73 | 0.71 | 0.08 | 0.2 | -0.54 | -0.69 | -0.47 | POL1 DNA REPLICATION |
| YKL113C | -1.12 | -0.45 | 0.29 | 0.79 | 0.3 | -0.04 | -0.56 | -0.79 | -0.9 | -0.71 | 0.24 | 0.55 | 0.5 | -0.27 | -0.18 | -0.25 | -0.89 | -0.56 | RAD27 DNA REPAIR S |
| YDL164C | -0.62 | -0.54 | 0.55 | 0.93 | 0.57 | -0.06 | -0.1 | -0.84 | -0.8 | -0.4 | 0.11 | 0.73 | 0.6 | -0.2 | -0.25 | -0.6 | -0.56 | -0.6 | CDC9 DNA REPLICATION |
| YGL038C | -0.86 | -0.22 | 0.5 | 0.57 | -0.4 | 0.06 | -0.69 | -0.43 | -0.4 | 0.2 | 0.32 | 0.63 | 0.31 | 0.14 | -0.1 | -0.12 | -0.45 | -0.32 | OCH1 PROTEIN GLYCOSYLA |
| YPL057C | 0.32 | -0.29 | 0.96 | 0.84 | 0.8 | 1.08 | 0.29 | -0.45 | -0.7 | 0.19 | 0.95 | 0.76 | 0.58 | 0.2 | 0.34 | -0.25 | -0.42 | -0.51 | SUR1 SPHINGOLIPID METAE |
| YKL067W | -0.51 | 0.21 | 0.45 | 1.03 | 0.77 | 0.93 | 0.29 | -0.12 | -0.4 | -0.3 | -0.3 | -0.03 | 0.37 | -0.14 | 0.16 | -0.23 | -0.25 | -0.74 | YNK1 NUCLEOTIDE METABC |
| YPR135W | -0.56 | -0.76 | 0.63 | 1.12 | 0.51 | -0.12 | -0.45 | -0.79 | -0.8 | -0.84 | 0.12 | 0.57 | 0.43 | -0.29 | -0.17 | -0.45 | -0.42 | -0.71 | CTF4 DNA REPLICATION |
| YDR309C | 0.53 | -0.62 | 0.33 | 0.38 | 0.11 | -0.74 | -1.09 | -1.06 | -0.5 | -0.3 | 1.52 | 0.59 | 0.64 | -0.3 | 0.53 | -0.17 | -0.79 | -0.42 | GIC2 BUD EMERGENCE |
| YGR152C | -0.49 | -0.58 | 0.8 | 0.84 | 0.57 | 0.34 | -0.01 | -0.42 | -0.5 | -0.38 | 0.43 | 0.55 | 0.42 | 0.21 | 0.04 | -0.3 | -0.17 | -0.71 | RSR1 BUD SITE SELECTION |
| YBL035C | -0.45 | -0.64 | 1.01 | 1.14 | 0.45 | -0.4 | -0.64 | 0.15 | -1.1 | 0.44 | 0.04 | 0.28 | 0.32 | 0.03 | -0.54 | -0.12 | -0.6 | -0.3 | POL12 DNA REPLICATION |
| YPR175W | -0.54 | -0.69 | 1.03 | 0.57 | 0.49 | -0.12 | -0.34 | -0.62 | -0.6 | -0.45 | 0.1 | 0.52 | 0.3 | -0.22 | -0.15 | -0.62 | -0.2 | -0.69 | DPB2 DNA REPLICATION |
| YER111C | -1.25 | -0.3 | 1.32 | 1.33 | 0.5 | 0.14 | -0.89 | -0.86 | -0.8 | 0.03 | 0.85 | 0.74 | 0.33 | -0.23 | -0.15 | -0.58 | -0.38 | -0.51 | SWI4 CELL CYCLE TR |

## Tab. 7

**Tab. 8**

| GenBank | | alpha 0 | alpha 7 | alpha 14 | alpha 21 | alpha 28 | alpha 35 | alpha 42 | alpha 49 | alpha 56 | alpha 63 | alpha 70 | alpha 77 | alpha 84 | alpha 91 | alpha 98 | alpha 10 | alpha 11 | alpha 119 |
|---|---|---|---|---|---|---|---|---|---|---|---|---|---|---|---|---|---|---|---|
| YPR120C | C30 | -0.92 | -0.32 | 0.98 | 1.03 | 0.32 | -0.03 | -0.12 | -0.34 | -0.29 | -0.27 | 0.76 | 0.67 | 0.37 | -0.17 | 0.16 | -0.14 | -0.15 | -0.43 |
| YJL115W | C30 | -0.32 | 0.49 | 0.61 | 1.43 | 0.58 | 0.3 | -0.45 | -0.42 | -0.06 | 0.06 | 0.34 | 0.58 | 0.36 | -0.1 | -0.32 | -0.14 | -0.42 | -0.38 |
| YCR065W | C30 | -1.22 | -0.23 | 0.54 | 0.66 | 0.18 | 0.07 | -0.69 | -0.47 | -0.43 | -0.6 | 0.18 | 0.77 | 0.66 | 0.38 | 0.1 | 0.28 | -0.4 | -0.38 |
| YDR097C | C26 | -0.56 | -0.69 | 0.7 | 1.2 | 1 | 0.4 | -0.47 | -0.67 | -0.2 | -0.54 | 1.16 | 1.24 | 0.81 | 0.34 | 0.11 | 0.19 | -0.6 | -0.49 |
| YKL045W | C30 | -1.03 | -0.22 | 0.63 | 0.61 | 0.29 | -0.09 | -0.62 | -0.86 | -1.03 | 0.19 | 0.65 | 0.53 | 0.24 | -0.49 | -0.32 | -0.45 | -0.64 | -1.43 |
| YNL262W | C30 | 0.84 | -0.51 | 0.49 | 0.58 | 0.87 | 0.24 | -0.18 | -0.64 | -0.43 | -0.49 | 0.03 | 0.32 | 0.43 | 0.08 | 0.04 | -0.56 | -0.32 | -0.71 |
| YOR074C | C26 | -1.43 | -0.6 | 0.28 | 0.79 | 0.88 | 0.28 | 0.01 | -1.03 | -0.97 | -0.4 | -0.67 | 0.45 | 0.44 | -0.2 | -0.56 | -0.51 | -0.92 | -1.09 |
| YER070W | C26 | -1.22 | -0.51 | 1.32 | 1.74 | 0.99 | 0.71 | -0.45 | -0.43 | -0.79 | -0.3 | 0.59 | 1.49 | 0.97 | 0.44 | 0.24 | 0.36 | -0.29 | -0.47 |
| YLR103C | C30 | -0.64 | -0.2 | 0.9 | 0.74 | 0.48 | 0.07 | -0.3 | -0.34 | -0.47 | -0.34 | 0.4 | 0.58 | 0.33 | -0.15 | -0.25 | -0.15 | -0.45 | -0.38 |
| YNL312W | C30 | -0.69 | -0.79 | 0.48 | 0.96 | 0.78 | 0.77 | 0.04 | -0.47 | -0.79 | -0.56 | 0.06 | 0.23 | 0.53 | -0.15 | 0.06 | -0.62 | -0.22 | -0.54 |
| YJL074C | C30 | -0.74 | -1.06 | 0.46 | 1.06 | 0.89 | 0.04 | -0.15 | -0.79 | -0.76 | -0.3 | 0.12 | 0.64 | 0.63 | -0.17 | -0.27 | -0.45 | -0.43 | -0.2 |
| YJL187C | C30 | -0.94 | -0.64 | -0.04 | 0.51 | 0.38 | -0.12 | -0.2 | -0.25 | -0.45 | -0.74 | 0.23 | 0.59 | 0.58 | 0.2 | 0.29 | 0.14 | -1.94 | -0.49 |
| YBR088C | C26 | -1.47 | -1.18 | 0.89 | 1.29 | 0.8 | -0.17 | -0.76 | 0.48 | -1.56 | -0.94 | 0.3 | 0.97 | 0.76 | -0.06 | -0.29 | -0.84 | -1.12 | -1.22 |
| YNL102W | C30 | -0.62 | 2.13 | 0.13 | 0.99 | 0.62 | -0.17 | -0.22 | -0.2 | -0.09 | -0.64 | 0.28 | 0.73 | 0.71 | 0.08 | 0.2 | -0.54 | -0.69 | -0.47 |
| YKL113C | C30 | -1.12 | -0.45 | 0.29 | 0.79 | 0.3 | -0.04 | -0.56 | -0.79 | -0.86 | -0.71 | 0.24 | 0.55 | 0.5 | -0.27 | -0.18 | -0.25 | -0.89 | -0.56 |
| YDL164C | C30 | -0.62 | -0.54 | 0.55 | 0.93 | 0.57 | -0.06 | -0.1 | -0.84 | -0.84 | -0.4 | 0.11 | 0.73 | 0.6 | -0.2 | -0.25 | -0.6 | -0.56 | -0.6 |
| YGL038C | C30 | -0.86 | -0.22 | 0.5 | 0.57 | -0.36 | 0.06 | -0.69 | -0.43 | -0.42 | 0.2 | 0.32 | 0.63 | 0.31 | 0.14 | -0.1 | -0.12 | -0.45 | -0.32 |
| YPL057C | C30 | 0.32 | -0.29 | 0.96 | 0.84 | 0.8 | 1.08 | 0.29 | -0.45 | -0.74 | 0.19 | 0.95 | 0.76 | 0.58 | 0.2 | 0.34 | -0.25 | -0.42 | -0.51 |
| YKL067W | C30 | -0.51 | 0.21 | 0.45 | 1.03 | 0.77 | 0.93 | 0.29 | -0.12 | -0.42 | -0.3 | -0.3 | -0.03 | 0.37 | -0.14 | 0.16 | -0.23 | -0.25 | -0.74 |
| YER001W | C26 | -2.18 | -0.58 | 0.87 | 1.71 | 0.64 | 0.66 | -0.27 | -0.43 | -0.97 | -0.84 | 0.18 | 1.46 | 1.13 | 1.1 | 0.31 | 0.07 | -0.86 | -0.76 |
| YPR135W | C30 | -0.56 | -0.76 | 0.63 | 1.12 | 0.51 | -0.12 | -0.45 | -0.79 | -0.76 | -0.84 | 0.12 | 0.57 | 0.43 | -0.29 | -0.17 | -0.45 | -0.42 | -0.71 |
| YOL007C | C26 | -1.43 | -1.25 | 0.83 | 0.73 | 0.77 | -0.47 | -0.32 | -1.18 | -1.47 | -0.71 | -0.32 | 0.58 | 0.78 | 0.39 | -0.27 | -0.4 | -0.84 | -1.03 |
| YPL256C | C26 | -1.69 | -0.97 | 1.11 | 1.69 | 0.45 | -0.07 | -0.64 | -1.6 | -1.79 | -1.36 | 0.07 | 1.29 | 0.82 | 0.28 | -0.1 | -0.6 | -0.67 | -1.32 |
| YIL140W | C26 | -1.43 | -1.03 | 1.37 | 0.74 | 0.26 | -0.17 | -0.84 | -1.18 | -1.09 | -1.03 | -0.45 | 0.7 | 0.29 | -0.36 | -0.32 | -0.51 | -0.6 | -1.32 |
| YDR309C | C30 | 0.53 | -0.62 | 0.33 | 0.38 | 0.11 | -0.74 | -1.09 | -1.06 | -0.47 | -0.3 | 1.52 | 0.59 | 0.64 | -0.3 | 0.53 | -0.17 | -0.79 | -0.42 |
| YMR199W | C26 | -1.6 | -0.97 | 1.25 | 0.83 | 0.9 | 0.44 | 0.03 | -0.58 | -1.15 | -0.81 | 0.62 | 1.1 | 0.95 | 0.26 | 0.31 | -0.06 | -0.45 | -0.92 |
| YGR152C | C30 | -0.49 | -0.58 | 0.8 | 0.84 | 0.57 | 0.34 | -0.01 | -0.42 | -0.47 | -0.38 | 0.43 | 0.55 | 0.42 | 0.21 | 0.04 | -0.3 | -0.17 | -0.71 |
| YBL035C | C30 | -0.45 | -0.64 | 1.01 | 1.14 | 0.45 | -0.4 | -0.64 | 0.15 | -1.09 | 0.44 | 0.04 | 0.28 | 0.32 | 0.03 | -0.54 | -0.12 | -0.6 | -0.3 |
| YPR175W | C30 | -0.54 | -0.69 | 1.03 | 0.57 | 0.49 | -0.12 | -0.34 | -0.62 | -0.56 | -0.45 | 0.1 | 0.52 | 0.3 | -0.22 | -0.15 | -0.62 | -0.2 | -0.69 |
| YER111C | C30 | -1.25 | -0.3 | 1.32 | 1.33 | 0.5 | 0.14 | -0.89 | -0.86 | -0.79 | 0.03 | 0.85 | 0.74 | 0.33 | -0.23 | -0.15 | -0.58 | -0.38 | -0.51 |

| P1 | P2 | P3 | P4 | P5 | P6 | OUT |
|---|---|---|---|---|---|---|
| 0.81 | 0 | 0.77 | 0.5 | 0.72 | 0.57 | 0.67 |

P1 = POSITIVE LINEAR CORRELATION COEFFICIENT
P2 = NEGATIVE LINEAR CORRELATION COEFFICIENT
P3 = QUADRATIC CORRELATION COEFFICIENT
P4 = PERCENTAGE OF GENES HAVING A FINAL VALUE LARGER THAN THE INITIAL VALUE
P5 = PERCENTAGE OF GENES HAVING THE SAME TIME EVOLUTION
P6 = PERCENTAGE OF GENES HAVING A MAXIMUM EXCURSION IN THE SAME TIME INSTANT

**Tab. 9**

| | alpha 0 | alpha 7 | alpha 14 | alpha 21 | alpha 28 | alpha 35 | alpha 42 | alpha 49 | alpha 56 | alpha 63 | alpha 70 | alpha 77 | alpha 84 | alpha 91 | alpha 98 | alpha 105 | alpha 112 | alpha 119 |
|---|---|---|---|---|---|---|---|---|---|---|---|---|---|---|---|---|---|---|
| YPR120C | 0 | 0.308 | 0.974 | 1 | 0.636 | 0.456 | 0.4103 | 0.297 | 0.323 | 0.3333 | 0.8615 | 0.815 | 0.662 | 0.3846 | 0.554 | 0.4 | 0.39487 | 0.2513 |
| YJL115W | 0.07 | 0.5 | 0.564 | 1 | 0.548 | 0.399 | 0 | 0.016 | 0.207 | 0.2713 | 0.4202 | 0.548 | 0.431 | 0.1862 | 0.069 | 0.1649 | 0.01596 | 0.0106 |
| YCR065W | 0 | 0.497 | 0.884 | 0.945 | 0.704 | 0.648 | 0.2663 | 0.377 | 0.397 | 0.3116 | 0.7035 | 1 | 0.945 | 0.804 | 0.663 | 0.7538 | 0.41206 | 0.4221 |
| YDR097C | 0.07 | 0 | 0.72 | 0.979 | 0.876 | 0.565 | 0.114 | 0.01 | 0.254 | 0.0777 | 0.9585 | 1 | 0.777 | 0.5337 | 0.415 | 0.456 | 0.04663 | 0.1036 |
| YKL045W | 0.19 | 0.582 | 0.99 | 0.981 | 0.827 | 0.644 | 0.3894 | 0.274 | 0.192 | 0.7788 | 1 | 0.942 | 0.803 | 0.4519 | 0.534 | 0.4712 | 0.37981 | 0 |
| YNL262W | 0.98 | 0.127 | 0.759 | 0.816 | 1 | 0.601 | 0.3354 | 0.044 | 0.177 | 0.1392 | 0.4684 | 0.652 | 0.722 | 0.5 | 0.475 | 0.0949 | 0.24684 | 0 |
| YOR074C | 0 | 0.359 | 0.74 | 0.961 | 1 | 0.74 | 0.6234 | 0.173 | 0.199 | 0.4459 | 0.329 | 0.814 | 0.81 | 0.5325 | 0.377 | 0.3983 | 0.22078 | 0.1472 |
| YER070W | 0 | 0.24 | 0.858 | 1 | 0.747 | 0.652 | 0.2601 | 0.267 | 0.145 | 0.3108 | 0.6115 | 0.916 | 0.74 | 0.5608 | 0.493 | 0.5338 | 0.31419 | 0.2534 |
| YLR103C | 0 | 0.286 | 1 | 0.896 | 0.727 | 0.461 | 0.2208 | 0.195 | 0.11 | 0.1948 | 0.6753 | 0.792 | 0.63 | 0.3182 | 0.253 | 0.3182 | 0.12338 | 0.1688 |
| YNL312W | 0.06 | 0 | 0.726 | 1 | 0.897 | 0.891 | 0.4743 | 0.183 | 0 | 0.1314 | 0.4857 | 0.583 | 0.754 | 0.3657 | 0.486 | 0.0971 | 0.32571 | 0.1429 |
| YJL074C | 0.15 | 0 | 0.717 | 1 | 0.92 | 0.519 | 0.4292 | 0.127 | 0.142 | 0.3585 | 0.5566 | 0.802 | 0.797 | 0.4198 | 0.373 | 0.2877 | 0.29717 | 0.4057 |
| YJL187C | 0.4 | 0.514 | 0.751 | 0.968 | 0.917 | 0.719 | 0.6877 | 0.668 | 0.589 | 0.4743 | 0.8577 | 1 | 0.996 | 0.8458 | 0.881 | 0.8221 | 0 | 0.5731 |
| YBR088C | 0.03 | 0.133 | 0.86 | 1 | 0.828 | 0.488 | 0.2807 | 0.716 | 0 | 0.2175 | 0.6526 | 0.888 | 0.814 | 0.5263 | 0.446 | 0.2526 | 0.15439 | 0.1193 |
| YNL102W | 0.02 | 1 | 0.312 | 0.596 | 0.465 | 0.184 | 0.1667 | 0.174 | 0.213 | 0.0177 | 0.344 | 0.504 | 0.496 | 0.273 | 0.316 | 0.0532 | 0 | 0.078 |
| YKL113C | 0 | 0.351 | 0.738 | 1 | 0.743 | 0.565 | 0.2932 | 0.173 | 0.136 | 0.2147 | 0.712 | 0.874 | 0.848 | 0.445 | 0.492 | 0.4555 | 0.12042 | 0.2932 |
| YDL164C | 0.12 | 0.169 | 0.785 | 1 | 0.797 | 0.441 | 0.4181 | 0 | 0 | 0.2486 | 0.5367 | 0.887 | 0.814 | 0.3616 | 0.333 | 0.1356 | 0.15819 | 0.1356 |
| YGL038C | 0 | 0.43 | 0.913 | 0.96 | 0.336 | 0.617 | 0.1141 | 0.289 | 0.295 | 0.7114 | 0.7919 | 1 | 0.785 | 0.6711 | 0.51 | 0.4966 | 0.27517 | 0.3624 |
| YPL057C | 0.58 | 0.247 | 0.934 | 0.868 | 0.846 | 1 | 0.5659 | 0.159 | 0 | 0.511 | 0.9286 | 0.824 | 0.725 | 0.5165 | 0.593 | 0.2692 | 0.17582 | 0.1264 |
| YKL067W | 0.13 | 0.537 | 0.672 | 1 | 0.853 | 0.944 | 0.5819 | 0.35 | 0.181 | 0.2486 | 0.2486 | 0.401 | 0.627 | 0.339 | 0.508 | 0.2881 | 0.27684 | 0 |
| YER001W | 0 | 0.411 | 0.784 | 1 | 0.725 | 0.73 | 0.491 | 0.45 | 0.311 | 0.3445 | 0.6067 | 0.936 | 0.851 | 0.8432 | 0.64 | 0.5784 | 0.33933 | 0.365 |
| YPR135W | 0.14 | 0.041 | 0.75 | 1 | 0.689 | 0.367 | 0.199 | 0.026 | 0.041 | 0 | 0.4898 | 0.719 | 0.648 | 0.2806 | 0.342 | 0.199 | 0.21429 | 0.0663 |
| YOL007C | 0.02 | 0.096 | 1 | 0.957 | 0.974 | 0.435 | 0.5 | 0.126 | 0 | 0.3304 | 0.5 | 0.891 | 0.978 | 0.8087 | 0.522 | 0.4652 | 0.27391 | 0.1913 |
| YPL256C | 0.03 | 0.236 | 0.833 | 1 | 0.644 | 0.494 | 0.3305 | 0.055 | 0 | 0.1236 | 0.5345 | 0.885 | 0.75 | 0.5948 | 0.486 | 0.342 | 0.32184 | 0.1351 |
| YIL140W | 0 | 0.143 | 1 | 0.775 | 0.604 | 0.45 | 0.2107 | 0.089 | 0.121 | 0.1429 | 0.35 | 0.761 | 0.614 | 0.3821 | 0.396 | 0.3286 | 0.29643 | 0.0393 |
| YDR309C | 0.62 | 0.18 | 0.544 | 0.563 | 0.46 | 0.134 | 0 | 0.011 | 0.238 | 0.3027 | 1 | 0.644 | 0.663 | 0.3027 | 0.621 | 0.3525 | 0.11494 | 0.2567 |
| YMR199W | 0 | 0.221 | 1 | 0.853 | 0.877 | 0.716 | 0.5719 | 0.358 | 0.158 | 0.2772 | 0.7789 | 0.947 | 0.895 | 0.6526 | 0.67 | 0.5404 | 0.40351 | 0.2386 |
| YGR152C | 0.14 | 0.084 | 0.974 | 1 | 0.826 | 0.677 | 0.4516 | 0.187 | 0.155 | 0.2129 | 0.7355 | 0.813 | 0.729 | 0.5935 | 0.484 | 0.2645 | 0.34839 | 0 |
| YBL035C | 0.29 | 0.202 | 0.942 | 1 | 0.691 | 0.309 | 0.2018 | 0.556 | 0 | 0.6861 | 0.5067 | 0.614 | 0.632 | 0.5022 | 0.247 | 0.435 | 0.21973 | 0.3543 |
| YPR175W | 0.09 | 0 | 1 | 0.733 | 0.686 | 0.331 | 0.2035 | 0.041 | 0.076 | 0.1395 | 0.4593 | 0.703 | 0.576 | 0.2733 | 0.314 | 0.0407 | 0.28488 | 0 |
| YER111C | 0 | 0.368 | 0.996 | 1 | 0.678 | 0.539 | 0.1395 | 0.151 | 0.178 | 0.4961 | 0.814 | 0.771 | 0.612 | 0.3953 | 0.426 | 0.2597 | 0.33721 | 0.2868 |

EP 1 452 993 A1

**TAB. 10**

| | |Δ1| | |Δ2| | |Δ3| | |Δ4| | |Δ5| | |Δ6| | |Δ7| | |Δ8| | |Δ9| | |Δ10| | |Δ11| | |Δ12| | |Δ13| | |Δ14| | |Δ15| | |Δ16| | |Δ17| |
|---|---|---|---|---|---|---|---|---|---|---|---|---|---|---|---|---|---|
| YPR120C | 0.30769 | 0.66667 | 0.02564 | 0.3641 | 0.17949 | 0.04615 | 0.11282 | 0.02564 | 0.01026 | 0.52821 | 0.04615 | 0.15385 | 0.27692 | 0.16923 | 0.15385 | 0.00513 | 0.14359 |
| YJL115V | 0.43085 | 0.06383 | 0.43617 | 0.45213 | 0.14894 | 0.39894 | 0.01596 | 0.19149 | 0.06383 | 0.14894 | 0.12766 | 0.11702 | 0.24468 | 0.11702 | 0.09574 | 0.14894 | 0.00532 |
| YCR065V | 0.49749 | 0.38693 | 0.0603 | 0.24121 | 0.05528 | 0.38191 | 0.11055 | 0.0201 | 0.08543 | 0.39196 | 0.29648 | 0.05528 | 0.1407 | 0.1407 | 0.09045 | 0.34171 | 0.01005 |
| YDR097C | 0.06736 | 0.72021 | 0.25907 | 0.10363 | 0.31088 | 0.45078 | 0.10363 | 0.24352 | 0.17617 | 0.88083 | 0.04145 | 0.2228 | 0.24352 | 0.11917 | 0.04145 | 0.40933 | 0.05699 |
| YKL045V | 0.38942 | 0.40865 | 0.00962 | 0.15385 | 0.18269 | 0.25481 | 0.11538 | 0.08173 | 0.58654 | 0.22115 | 0.05769 | 0.13942 | 0.35096 | 0.08173 | 0.0625 | 0.09135 | 0.37981 |
| YNL262V | 0.85443 | 0.63291 | 0.05696 | 0.18354 | 0.39873 | 0.26582 | 0.29114 | 0.13291 | 0.03797 | 0.32911 | 0.18354 | 0.06962 | 0.22152 | 0.02532 | 0.37975 | 0.1519 | 0.24684 |
| YOR074C | 0.35931 | 0.38095 | 0.22078 | 0.03896 | 0.25974 | 0.11688 | 0.45022 | 0.02597 | 0.24675 | 0.11688 | 0.48485 | 0.00433 | 0.27706 | 0.15584 | 0.02165 | 0.17749 | 0.07359 |
| YER070V | 0.23986 | 0.61824 | 0.14189 | 0.25338 | 0.09459 | 0.39189 | 0.00676 | 0.12162 | 0.16554 | 0.30068 | 0.30405 | 0.17568 | 0.17905 | 0.06757 | 0.04054 | 0.21959 | 0.06081 |
| YLR103C | 0.28571 | 0.71429 | 0.1039 | 0.16883 | 0.26623 | 0.24026 | 0.02597 | 0.08442 | 0.08442 | 0.48052 | 0.11688 | 0.16234 | 0.31169 | 0.06494 | 0.06494 | 0.19481 | 0.04545 |
| YNL312V | 0.05714 | 0.72571 | 0.27429 | 0.10286 | 0.00571 | 0.41714 | 0.29143 | 0.18286 | 0.13143 | 0.35429 | 0.09714 | 0.17143 | 0.38857 | 0.12 | 0.38857 | 0.22857 | 0.18286 |
| YJL074C | 0.15094 | 0.71698 | 0.28302 | 0.08019 | 0.40094 | 0.08962 | 0.30189 | 0.01415 | 0.21698 | 0.19811 | 0.24528 | 0.00472 | 0.37736 | 0.04717 | 0.08491 | 0.00943 | 0.10849 |
| YJL187C | 0.11858 | 0.23715 | 0.21739 | 0.05138 | 0.19763 | 0.03162 | 0.01976 | 0.07905 | 0.11462 | 0.3834 | 0.14229 | 0.00395 | 0.1502 | 0.03557 | 0.05929 | 0.82213 | 0.57312 |
| YBR088C | 0.10175 | 0.72632 | 0.14035 | 0.17193 | 0.34035 | 0.20702 | 0.43509 | 0.71579 | 0.21754 | 0.43509 | 0.23509 | 0.07368 | 0.28772 | 0.0807 | 0.19298 | 0.09825 | 0.03509 |
| YNL102V | 0.97518 | 0.68794 | 0.28369 | 0.13121 | 0.28014 | 0.01773 | 0.00709 | 0.03901 | 0.19504 | 0.32624 | 0.15957 | 0.00709 | 0.2234 | 0.04255 | 0.26241 | 0.05319 | 0.07801 |
| YKL113C | 0.35079 | 0.38743 | 0.26178 | 0.25654 | 0.17801 | 0.27225 | 0.12042 | 0.03665 | 0.07853 | 0.49738 | 0.1623 | 0.02618 | 0.40314 | 0.04712 | 0.03665 | 0.33508 | 0.17277 |
| YDL164C | 0.0452 | 0.61582 | 0.21469 | 0.20339 | 0.35593 | 0.0226 | 0.41808 | 0 | 0.24859 | 0.28814 | 0.35028 | 0.07345 | 0.45198 | 0.02825 | 0.19774 | 0.0226 | 0.0226 |
| YGL038C | 0.42953 | 0.48322 | 0.04698 | 0.62416 | 0.28188 | 0.50336 | 0.1745 | 0.00671 | 0.41611 | 0.08054 | 0.20805 | 0.21477 | 0.11409 | 0.16107 | 0.01342 | 0.22148 | 0.08725 |
| YPL057C | 0.33516 | 0.68681 | 0.06593 | 0.02198 | 0.15385 | 0.43407 | 0.40659 | 0.15934 | 0.51099 | 0.41758 | 0.1044 | 0.0989 | 0.20879 | 0.07692 | 0.32418 | 0.09341 | 0.04945 |
| YKL067V | 0.40678 | 0.13559 | 0.32768 | 0.14689 | 0.0904 | 0.36158 | 0.23164 | 0.16949 | 0.0678 | 0 | 0.15254 | 0.22599 | 0.28814 | 0.16949 | 0.22034 | 0.0113 | 0.27684 |
| YER001V | 0.41131 | 0.37275 | 0.21594 | 0.27506 | 0.00514 | 0.23907 | 0.04113 | 0.13882 | 0.03342 | 0.26221 | 0.32905 | 0.08483 | 0.00771 | 0.20308 | 0.0617 | 0.23907 | 0.02571 |
| YPR135V | 0.10204 | 0.70918 | 0.25 | 0.31122 | 0.32143 | 0.16837 | 0.17347 | 0.01531 | 0.04082 | 0.4898 | 0.22959 | 0.07143 | 0.36735 | 0.06122 | 0.14286 | 0.01531 | 0.14796 |
| YOL007C | 0.07826 | 0.90435 | 0.04348 | 0.01739 | 0.53913 | 0.06522 | 0.37391 | 0.12609 | 0.33043 | 0.16957 | 0.3913 | 0.08696 | 0.16957 | 0.28696 | 0.05652 | 0.1913 | 0.08261 |
| YPL256C | 0.2069 | 0.5977 | 0.16667 | 0.35632 | 0.14943 | 0.16379 | 0.27586 | 0.0546 | 0.12356 | 0.41092 | 0.35057 | 0.13506 | 0.15517 | 0.1092 | 0.14368 | 0.02011 | 0.18678 |
| YIL140V | 0.14286 | 0.85714 | 0.225 | 0.17143 | 0.15357 | 0.23929 | 0.12143 | 0.03214 | 0.02143 | 0.20714 | 0.41071 | 0.14643 | 0.23214 | 0.01429 | 0.06786 | 0.03214 | 0.25714 |
| YDR309C | 0.44061 | 0.36398 | 0.01916 | 0.10345 | 0.32567 | 0.1341 | 0.01149 | 0.22605 | 0.06513 | 0.69732 | 0.35632 | 0.01916 | 0.36015 | 0.31801 | 0.2682 | 0.23755 | 0.14176 |
| YMR199V | 0.22105 | 0.77895 | 0.14737 | 0.02456 | 0.1614 | 0.14386 | 0.21404 | 0.2 | 0.1193 | 0.50175 | 0.16842 | 0.05263 | 0.24211 | 0.01754 | 0.12982 | 0.13684 | 0.16491 |
| YGR152C | 0.05806 | 0.89032 | 0.02581 | 0.17419 | 0.14839 | 0.22581 | 0.26452 | 0.03226 | 0.05806 | 0.52258 | 0.07742 | 0.08387 | 0.13548 | 0.10968 | 0.21935 | 0.08387 | 0.34839 |
| YBL035C | 0.0852 | 0.73991 | 0.0583 | 0.30942 | 0.38117 | 0.10762 | 0.35426 | 0.55605 | 0.6861 | 0.17937 | 0.10762 | 0.01794 | 0.13004 | 0.25561 | 0.18834 | 0.21525 | 0.13453 |
| YPR175V | 0.08721 | 1 | 0.26744 | 0.04651 | 0.35465 | 0.12791 | 0.16279 | 0.03488 | 0.06395 | 0.31977 | 0.24419 | 0.12791 | 0.30233 | 0.0407 | 0.27326 | 0.24419 | 0.28488 |
| YER111C | 0.36822 | 0.62791 | 0.00388 | 0.32171 | 0.13953 | 0.39922 | 0.01163 | 0.02713 | 0.31783 | 0.31783 | 0.04264 | 0.15891 | 0.21705 | 0.03101 | 0.16667 | 0.07752 | 0.05039 |

**European Patent Office**

**EUROPEAN SEARCH REPORT**

Application Number

EP 02 42 5791

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GETZ G LEVINE E DOMANY E: "Coupled two-way clustering analysis of gene microarray data" PROCEEDINGS OF THE NATIONAL ACADEMY OF SCIENCES OF USA, NATIONAL ACADEMY OF SCIENCE. WASHINGTON, US, vol. 97, no. 22, 24 October 2000 (2000-10-24), pages 12079-12084, XP002953907 ISSN: 0027-8424 | 1,2,4-13 | G06F19/00 |
| Y | * abstract * <br> * page 12079, left-hand column, paragraph 1 - page 12080, left-hand column, paragraph 6 * <br> * page 12080, right-hand column, paragraph 7 * <br> * page 12081, left-hand column, paragraph 3 * <br> * page 12081, right-hand column, paragraph 2 - page 12082, left-hand column, paragraph 4 * <br> * page 12082, left-hand column, paragraph 7 - right-hand column, paragraph 5 * <br> * page 12083, left-hand column, paragraph 2 - right-hand column, paragraph 5 * | 3,14 | |
| X | WO 02 093453 A (MILLER BRETT N ;MINE INC X (US); RAY SANDIP (US); HYTOPOULOS EVANG) 21 November 2002 (2002-11-21) * abstract * * paragraphs '0003!,'0026!,'0045!-'0048!,'0055!,'0068!,'0076! * * figures 2,4,9 * * claims 1-3,11,15,16,20,21,29,30,44,45 * <br> -/-- | 1,2,4,7,13 | |

TECHNICAL FIELDS SEARCHED (Int.Cl.7)

G06F
C12Q
G06N

The present search report has been drawn up for all claims

| Place of search | Date of completion of the search | Examiner |
|---|---|---|
| THE HAGUE | 27 November 2003 | Swarén, P. |

**European Patent Office**

# EUROPEAN SEARCH REPORT

Application Number

EP 02 42 5791

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | D'HAESELEER P ET AL: "GENETIC NETWORK INFERENCE: FROM CO-EXRESSION CLUSTERING TO REVERSE ENGINEERING" BIOINFORMATICS, OXFORD UNIVERSITY PRESS, OXFORD, GB, vol. 16, no. 8, August 2000 (2000-08), pages 707-726, XP001088339 ISSN: 1367-4803 | 3,14 | |
| A | cited in the application * abstract * * page 710, left-hand column, paragraph 4 - page 715, left-hand column, paragraph 2 * * page 717, right-hand column, paragraph 2 * * page 718, right-hand column, paragraph 2 * * page 719, right-hand column, paragraph 4 - page 721, left-hand column, paragraph 1 * --- | 1,2,5-13 | |
| A | TAVAZOIE ET AL: "Systematic determination of genetic network architecture" NATURE GENETICS, NATURE AMERICA, NEW YORK, US, vol. 22, July 1999 (1999-07), pages 281-285, XP002953903 ISSN: 1061-4036 * the whole document * --- -/-- | 2,4,5,7, 10-13 | TECHNICAL FIELDS SEARCHED (Int.Cl.7) |

The present search report has been drawn up for all claims

| Place of search | Date of completion of the search | Examiner |
|---|---|---|
| THE HAGUE | 27 November 2003 | Swarén, P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

EP 1 452 993 A1

European Patent
Office

**EUROPEAN SEARCH REPORT**

Application Number

EP 02 42 5791

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | TAMAYO P ET AL: "INTERPRETING PATTERNS OF GENE EXPRESSION WITH SELF-ORGANIZING MAPS:METHODS AND APPLICATION TO HEMATOPOIETIC DIFFERENTIATION" PROCEEDINGS OF THE NATIONAL ACADEMY OF SCIENCES OF USA, NATIONAL ACADEMY OF SCIENCE. WASHINGTON, US, vol. 96, March 1999 (1999-03), pages 2907-2912, XP000942169 ISSN: 0027-8424 * abstract * * page 2907, left-hand column, paragraph 2 - page 2908, left-hand column, paragraph 5 * * page 2908, right-hand column, paragraph 2 * * page 2909, left-hand column, paragraph 2 - page 2910, right-hand column, paragraph 2 * * figures 2-4 * | 2,5, 10-12 | |
| A | US 2002/156587 A1 (WOOLF PETER JAMES ET AL) 24 October 2002 (2002-10-24) * abstract * * paragraphs '0003!-'0009!,'0022!,'0037!-'0043!,'0046!, '0048!,'0057!-'0059!,'0139!,'0172! * * figures 5,6,11 * * claims 1-4,16 * | 3,8,14 | TECHNICAL FIELDS SEARCHED (Int.Cl.7) |

The present search report has been drawn up for all claims

| Place of search | Date of completion of the search | Examiner |
|---|---|---|
| THE HAGUE | 27 November 2003 | Swarén, P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**ANNEX TO THE EUROPEAN SEARCH REPORT
ON EUROPEAN PATENT APPLICATION NO.** EP 02 42 5791

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2003

| Patent document cited in search report | Publication date | Patent family member(s) | Publication date |
|---|---|---|---|
| WO 02093453 A | 21-11-2002 | WO 02093453 A2 | 21-11-2002 |
| US 2002156587 A1 | 24-10-2002 | NONE | |

EPO FORM P0459

For more details about this annex : see Official Journal of the European Patent Office, No. 12/82